(19)

Europäisches Patentamt

European Patent Office

Office européen des brevets

(11)  **EP 4 557 297 A1**

(12)  **EUROPEAN PATENT APPLICATION**

(43) Date of publication:
21.05.2025  Bulletin 2025/21

(51) International Patent Classification (IPC):
*G16B 20/00* (2019.01)    *G16B 40/20* (2019.01)

(21) Application number: 24213383.3

(22) Date of filing: 15.11.2024

(52) Cooperative Patent Classification (CPC):
G16B 40/20; G16B 20/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority:  15.11.2023  US 202363599471 P

(71) Applicant: Tempus AI, Inc.
Chicago, IL 60654 (US)

(72) Inventors:
• Ahmed, Talal
Highland Park, 08904 (US)
• Pelossof, Raphael
New York, 10044 (US)
• Carty, Mark
Brooklyn, 11203 (US)

(74) Representative: Hofmann, Matthias
Boehmert & Boehmert
Anwaltspartnerschaft mbB
Pettenkoferstraße 22
80336 München (DE)

(54)  **METHODS AND SYSTEMS FOR STRATIFYING PATIENT CANCER RISK USING COMPUTATIONAL ONCOLOGY AND MOLECULAR DATA**

(57)     A implemented method, computing system and computer-readable medium for stratifying patient cancer risk using molecular data includes receiving molecular data; processing the molecular data using a machine learning model; and generating a matched treatment strategy for the patient based upon the patient's molecular data risk. A computer-implemented method, computing system and computer-readable medium for training a machine learning model to stratify patient cancer risk using molecular data includes receiving a patient training dataset, and a reference training dataset; selecting a cohort of patients; selecting a small subset of genes using univariate selection; generating a corrected reference training dataset; selecting a smaller subset of genes using multivariate selection; training a survival model; and (g) selecting a decision threshold to identify a patient population.

FIG. 1

EP 4 557 297 A1

**Description**

**CROSS-REFERENCE TO RELATED APPLICATIONS**

The present application claims priority to U.S. Patent Application No. 63/599,471, entitled METHODS AND SYSTEMS FOR STRATIFYING PATIENT CANCER RISK USING COMPUTATIONAL ONCOLOGY AND MOLECULAR DATA, filed on November 15, 2023, and hereby incorporated by reference in its entirety.

**FIELD**

The present disclosure is directed to methods and systems for stratifying patient cancer risk using computational oncology and molecular data, and more particularly, to techniques for training and operating one or more machine learning models to process molecular data of a patient to prognosticate the patient's cancer risk profile.

**BACKGROUND**

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Conventional cancer diagnosis and treatment models (e.g., simple classifiers) suffer from several drawbacks. For example, such models require rigidly uniform input data (e.g., censored patient data often cannot be used for training), and any deviations in the availability of training data may cause conventional modeling to be inaccurate.

For at least one type of cancer and corresponding therapy decision-making, clinicians are currently struggling to understand a clinically homogeneous group of patients that do not present with symptoms or conditions sufficient to allow the clinician to adequately diagnose the patient's risk profile based upon clinical criteria. Specifically, for certain patients, it is not always clear whether a clinician should select a more aggressive treatment for the patient (for example, the patient has a worse prognosis and higher risk) or less aggressive treatment for the patient (for example, the patient has a better prognosis and lower risk).

As an example cancer type and corresponding therapy decision-making, endometrioid endometrial cancer adjuvant therapy decisions currently rely on risk stratification using clinical features such as histology, grade, stage, and lymphovascular space invasion (LVSI). Recently, molecular classification systems originating from TCGA, evaluated in GOG-210 and PORTEC-3 defined four prognostic subtypes based on POLE, MSI-H/MMR-D, and p53 alterations. Although valuable, this molecular approach still has significant limitations such as applicability to the majority of EEC patients categorized as no-specific molecular profile (NSMP) and the potential need to resolve pathogenic and prognostic heterogeneity within MMR-D, and TP53 subtypes.

A high to medium risk group may include a not insignificant percentage of patients (e.g., 9%) that will have a distant recurrence of cancer, and would benefit from early treatment. While some of the patients in this group may benefit from early intervention treatment, the decision of whether to treat these patients early is opaque, given the aforementioned homogeneity.

Many of these patients will undergo resection, and receive adjuvant therapy, while recovering at home. However, many early stage cases that lack metastasis look essentially the same from a clinical perspective, and clinicians do not know whether to escalate, deescalate or maintain the patient's treatment strategy. This can lead directly to overtreatment, when the patient is actually low risk, or undertreatment (a certain proportion of these cases eventually undergo a distant recurrence). Further, in general, conventional techniques have not adequately systematized cohort selection.

Accordingly, there is an opportunity for improved platforms and technologies for stratifying patient cancer risk using computational oncology and molecular data, by enhancing data availability, systematizing cohort selection criteria and proactively determining patient prognostics.

**SUMMARY**

In an aspect, a computer-implemented method for stratifying patient cancer risk using molecular data includes (a) receiving, via one or more processors, molecular data corresponding to a patient; (b) processing, via one or more processors, the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and (c) generating a matched treatment strategy corresponding to the patient based upon the patient's molecular data risk.

In another aspect, a computer-implemented method for training a machine learning model to stratify patient cancer risk using molecular data includes (a) receiving, via one or more processors: (i) a patient training dataset, wherein the patient training dataset includes respective molecular data of a plurality of patients, and (ii) a reference training dataset including respective molecular data of a plurality of patients; (b) selecting, via one or more processors, a cohort of patients from the patient training dataset; (c) selecting, via one or more processors, a small subset of genes from the patient training dataset using univariate gene selection; (d) generating a corrected reference training dataset by processing the reference training dataset to correct biases in the molecular data of the plurality of patients; (e) selecting, via one or more processors, a smaller subset of genes from the small subset of genes, using multivariate gene selection; (f) training, via one or more processors, a survival model, wherein the training includes determining a set of hyperparameters; and (g) selecting, via one or more processors, a decision threshold to identify a patient population having an RNA risk profile.

In yet another aspect, a computing system includes one or more processors, and one or more memories having stored thereon computer-executable instructions that, when executed by the one or more processors, cause the computing system to: (a) receive molecular data corresponding to a patient; (b) process the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and (c) generate a matched treatment strategy corresponding to the patient based upon the patient's molecular data risk.

In still another aspect, a computing system includes one or more processors, and one or more memories having stored thereon computer-executable instructions that, when executed by the one or more processors, cause the computing system to: (a) receive: (i) a patient training dataset, wherein the patient training dataset includes respective molecular data of a plurality of patients, and (ii) a reference training dataset including respective molecular data of a plurality of patients; (b) select a cohort of patients from the patient training dataset; (c) select a small subset of genes from the patient training dataset using univariate gene selection; (d) generate a corrected reference training dataset by processing the reference training dataset to correct biases in the molecular data of the plurality of patients; (e) select a smaller subset of genes from the small subset of genes, using multivariate gene selection; (f) train a survival model, wherein the training includes determining a set of hyperparameters; and (g) select a decision threshold to identify a patient population having an RNA risk profile.

In another aspect, a computer-readable medium includes computer-executable instructions that, when executed by one or more processors, cause a computer to: (a) receive molecular data corresponding to a patient; (b) process the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and (c) generate a matched treatment strategy corresponding to the patient based upon the patient's molecular data risk.

In yet another aspect, a computer-readable medium includes computer-executable instructions that, when executed by one or more processors, cause a computer to: (a) receive: (i) a patient training dataset, wherein the patient training dataset includes respective molecular data of a plurality of patients, and (ii) a reference training dataset including respective molecular data of a plurality of patients; (b) select a cohort of patients from the patient training dataset; (c) select a small subset of genes from the patient training dataset using univariate gene selection; (d) generate a corrected reference training dataset by processing the reference training dataset to correct biases in the molecular data of the plurality of patients; (e) select a smaller subset of genes from the small subset of genes, using multivariate gene selection; (f) train a survival model, wherein the training includes determining a set of hyperparameters; and (g) select a decision threshold to identify a patient population having an RNA risk profile.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

The figures described below depict various aspects of the system and methods disclosed herein. It should be understood that each figure depicts an example of aspects of the present systems and methods.

FIG. 1 depicts an exemplary computing system for stratifying patient cancer risk using molecular data by training and operating machine learning models, according to some aspects.

FIG. 2 depicts an exemplary computer-implemented machine learning model training method, according to some aspects.

FIG. 3 depicts an exemplary computer-implemented cohort selection method for endometrioid endometrial cancer, according to some aspects.

FIG. 4 depicts an exemplary patient risk group vs. treatment strategy table, according to some aspects.

FIG. 5A depicts a heatmap showing the clustering of a final gene set, according to some aspects.

FIG. 5B depicts a plot of Kaplan-Meier-adjusted event risk rates, wherein the x-axis corresponds to the percentile on the TCGA-stage12 log-partial hazard scores for the decision threshold

FIG. 5C depicts a survival plot for TCGA-stage12 patients, according to some aspects.

FIG. 6A depicts exemplary volcano plots comparing proprietary high-intermediate risk and TCGA high-intermediate risk and stage 1+2 subsets, according to some aspects.

FIG. 6B depicts exemplary scatter plots comparing proprietary high-intermediate risk and TCGA high-intermediate risk and stage 1+2 subsets, according to some aspects.

FIG. 6C depicts exemplary volcano plots comparing proprietary high-intermediate risk and TCGA high-intermediate risk and stage 1+2 subsets for primary tumors, according to some aspects.

FIG. 6D depicts exemplary scatter plots comparing proprietary high-intermediate risk and TCGA high-intermediate risk and stage 1+2 subsets for primary tumors, according to some aspects.

FIG. 6E depicts exemplary volcano plots comparing proprietary high-intermediate risk and Institutional-not-high-intermediate risk and stage 1+2 subsets, according to some aspects.

FIG. 6F depicts exemplary scatter plots comparing proprietary high-intermediate risk and Institutional-not-high-intermediate risk and stage 1+2 subsets, according to some aspects.

FIG. 7 depicts an exemplary digital report of a patient's molecular risk evaluation and prediction of disease recurrence from a pathology analysis, according to some aspects.

## DETAILED DESCRIPTION

### Overview

The present techniques are directed to methods and systems for stratifying patient cancer risk using molecular data, and more particularly, to techniques for training and operating one or more machine learning models to process molecular data of a patient to prognosticate the patient's cancer risk profile.

Conventional diagnosis and treatment of cancer patients depends on classic histopathological and immunohistochemical techniques. The molecular characterization of tumor samples provides additional insight on cancer biology beyond the classical clinical factors like stage, grade, histology, etc. The present techniques may generate additional information and identify patient tumors with negative prognosis from within a group of patients that are known to be comparable, in terms of clinical factors, for the risk of disease progression. The present techniques may further generate recommendations regarding more (or less) aggressive treatments for these patients.

In some examples, a predicted prognosis or risk profile of a patient, as determined by processing molecular data using a machine learning model, may be utilized to ascertain whether the patient aligns with inclusion or exclusion criteria for a specific trial. Alternatively, the predicted prognoses or risk profiles of numerous patients within a database may be employed to estimate the number of patients who might be suitable candidates for a particular trial. The machine learning model employed for these purposes may be trained using a patient training dataset and a reference training dataset. This model is capable of employing univariate gene selection, RNA bias correction, and multivariate gene selection to filter and correct its training data. Additionally, the model may incorporate a survival model. Based on the patient's molecular data risk, a matched treatment strategy may be generated, which may include, but is not limited to, systemic therapy, external beam radiation therapy, brachytherapy, or observation. This approach allows for a more nuanced understanding of patient risk profiles based on molecular data, which can be particularly useful in stratifying patients within clinical risk groups such as low clinical risk, low-intermediate clinical risk, high-intermediate clinical risk, or high clinical risk. For instance, patients within the high-intermediate clinical risk group who are determined to have a high molecular risk may be matched with more aggressive treatment strategies such as systemic therapy or external beam radiation therapy. Conversely, those within the same clinical risk group but with a low molecular risk may be matched with observation instead, potentially avoiding unnecessary treatments that could have adverse effects. The method and systems described herein offer a significant advancement in the field of patient care and clinical trial selection, leveraging the power of machine learning and molecular data to make more informed decisions regarding patient treatment strategies and trial inclusion.

That is, the present molecular profiling techniques may also (or alternatively) be used in computational oncology applications to identify patients with low risk of disease progression from within the group of patients with similar clinical profile. These patients with good prognosis, as identified by molecular data, can be recommended for treatment escalation.

Data generated by assays such as a targeted oncology DNA sequencing panel (also referred to as Tempus xT Solid Tumor + Normal Match or xT) and/or a whole transcriptome RNA seq panel (also referred to as xR or Tempus xR), for example, may be used to train and operate the prognostic models discussed herein. In particular, RNA seq data from xR may be used to predict risk of disease progression. Some relevant events of interest that characterize risk of disease progression include progression-free survival (PFS), disease-free survival (DFS), recurrence-free survival (RFS) and overall survival (OS).

Building prognostic models on RNA expression (xR) datasets is non-trivial because of technical challenges like patient selection (for training), biomarker identification, survival modeling, and selection of an operating point (i.e., decision threshold that segregates patients with good prognosis and bad prognosis, where threshold is applied on output of the survival model). Moreover, for a given cancer type and indication of interest, a data set having a good representation of the true patient population as seen in the clinic may not be available, so it may not always be feasible to train prognostic models on in-house patient data, or to adequately validate such trained models (e.g., due to a lack of sufficient held-out data to form an independent validation dataset for validation of such prognostic models).

To solve this problem, the present techniques may include machine learning techniques for building in-house proprietary RNA-based prognostic models, trained on external, publicly available RNA expression datasets with clinical outcomes. Because these models are trained on external datasets, relevant Tempus data can be used for held-out independent validation. Even when relevant clinical outcomes are not available at Tempus, or the Tempus patient population is not representative of the true clinical population, the present machine learning techniques can be used for training RNA-based prognostic models on external RNA datasets, such that these models can be evaluated on the samples from the xR assay at evaluation time. When training early stage cancer progression models in particular, the externally available datasets tend to be more heterogeneous in terms of patient populations, resulting in them being a good representation of the patient population, which results in training of robust and reproducible RNA-based prognostic models.

The present techniques may be used to identify matching patient populations between a cohort (e.g., a The Cancer Genome Atlas (TCGA) data cohort) and the Tempus cohort. The present techniques may be used to perform patient selection based on molecular information, in contrast to the prevailing conventional method of selecting patient population according to clinical criteria. A limitation of the clinical-patient-selection paradigm is that it does not take into account the molecular diversity of the underlying selected population. Understanding and controlling molecular diversity is important for success in creating molecular algorithms that are robust and can validate on multiple cohorts.

One example of molecular cohort selection discussed herein as a motivating example is that of endometrioid endometrial cancer. Specifically, the present machine learning techniques may be used to train an RNA-based prognostic model for risk stratification of high-intermediate risk endometrioid endometrial cancer patients.

When looking at molecular predictors of response to therapy, it was observed empirically that in TCGA, a different set of genes is predictive than the set found in the proprietary database. A greedy algorithm was used to programmatically update clinical criteria of the proprietary database funnel and ultimately, the present techniques identified a molecular match between proprietary database and TCGA (predominantly involving exclusion of uterine sarcomas). This allowed a prognosticator to be trained on TCGA data, and then validated on proprietary database data.

Specifically, the present techniques may include training a prognostic gene signature in a cross-validation experimental setting on an Endometrial Carcinoma TCGA cohort. This may include modeling a log rank p-value of 0.078, median survival difference of 12 months, and Hazard ratio of 2.82 on proprietary database stage I+II patients. In this example, proprietary database PFS data may not be used for model training. The larger TCGA cohort may be used instead for model training. The prognostic model may be validated on (i) the corrected proprietary database v1 cohort and (ii) the proprietary database v2 cohort, none of which were used in training. The training cohort (TCGA) may be was corrected to proprietary database RNA v2, and thus, the prognostic model may be native to RNA v2. Because the prognostic model is native to RNA v2, it can make risk predictions on future RNA v2 samples without modification.

The above-described training is highly advantageous, and improves prognosticator methods by enabling more data sets to be used. And further, as described above, by training on an external data source, internally-generated data may be used for validation. This is a particularly important result, because training may requires significantly more data than validation (e.g., three times as much data for training than for validation, or more). In short, this advance makes conventionally training-infeasible algorithms now feasible.

On the other hand, conventional works had to carefully curate an in-house training cohort for training an in-house cancer prognosis model. In the present techniques, however, publicly available data may be used to train an in-house prognostic model. This enables significant computational resources that would otherwise be required for sequencing and curating of a training cohort. The in-house proprietary database RNA dataset can be leveraged for independent held out validation. Moreover, the training strategy leads to robust and reproducible RNA-based prognostic models, because the present techniques may (i) train on larger publicly available datasets, and (ii) select biomarkers (e.g., genes) in a multi-stage, cascaded model setup.

## Exemplary Computing Environments

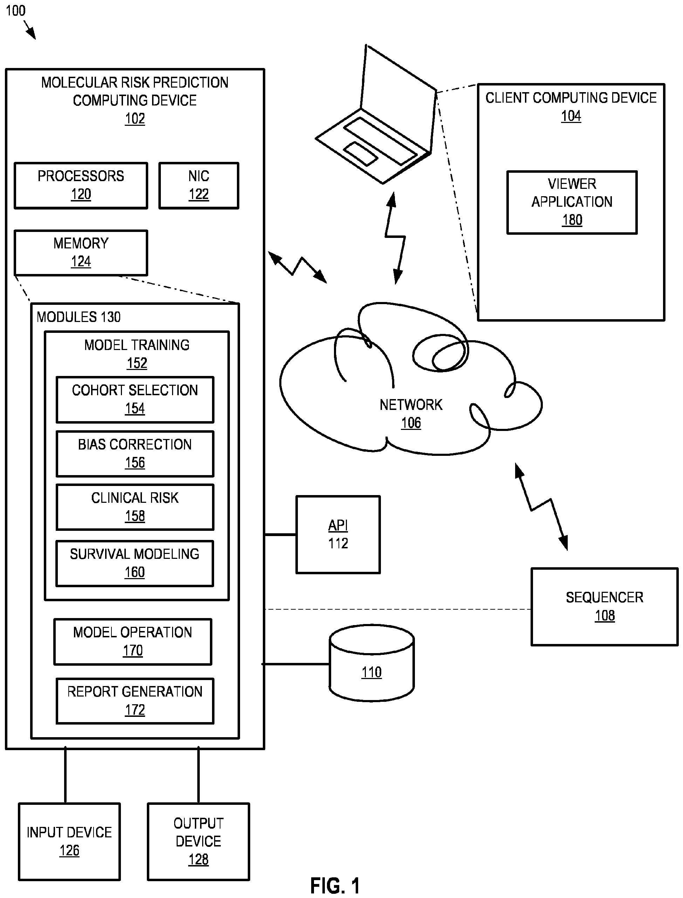

FIG. 1 illustrates an exemplary computing environment 100 for performing the present techniques, according to some aspects. The environment 100 may include computing resources for stratifying patient cancer risk using computational oncology and molecular data, and more particularly, for processing RNA data using machine learning to train models to prognostically classify patient molecular risk as high or low, and/or for further processing/ computations based on such classifications, such as recommendations and/or reports regarding treatment strategies.

The computing environment 100 may include a molecular risk status prediction computing device 102, a client computing device 104, an electronic network 106, a sequencer system 108 and an electronic database 110. The molecular risk status prediction computing device 102 may include an application programming interface 112 that enables programmatic access to the molecular risk status prediction computing device 102. The components of the computing environment 100 may be communicatively connected to one another via the electronic network 106, in some aspects. Each will now be described in greater detail.

The molecular risk status prediction computing device 102 may implement, *inter alia,* training and operation of machine learning models for predicting molecular cancer risk of one or more patients, patient identification and report generation. In some aspects, the molecular risk status prediction computing device 102 may be implemented as one or more computing devices (e.g., one or more servers, one or more laptops, one or more mobile computing devices, one or more tablets, one or more wearable devices, one or more cloud-computing virtual instances, etc.). The molecular risk status prediction computing device 102 may include one or more processors 120, one or more network interface controllers 122, one or more memories 124, an input device 126 and an output device 128.

In some aspects, the one or more processors 120 may include one or more central processing units, one or more graphics processing units, one or more field-programmable gate arrays, one or more application-specific integrated circuits, one or more tensor processing units, one or more digital signal processors, one or more neural processing units, one or more RISC-V processors, one or more coprocessors, one or more specialized processors/accelerators for artificial intelligence or machine learning-specific applications, one or more microcontrollers, etc.

The molecular risk status prediction computing device 102 may include one or more network interface controllers 122, such as Ethernet network interface controllers, wireless network interface controllers, etc. The network interface controllers 122 may include advanced features, in some aspects, such as hardware acceleration, specialized networking protocols, etc.

The memories 124 of the molecular risk status prediction computing device 102 may include volatile and/or non-volatile storage media. For example, the memories 124 may include one or more random access memories, one or more read-only memories, one or more cache memories, one or more hard disk drives, one or more solid-state drives, one or more non-volatile memory express, one or more optical drives, one or more universal serial bus flash drives, one or more external hard drives, one or more network-attached storage devices, one or more cloud storage instances, one or more tape drives, etc.

The memories 124 may have stored thereon one or more modules 130, for example, as one or more sets of computer-executable instructions. In some aspects, the modules 130 may include additional storage, such as one or more operating systems (e.g., Microsoft Windows, GNU/Linux, Mac OSX, etc.). The operating systems may be configured to run the modules 130 during operation of the molecular risk status prediction computing device 102 - for example, the modules 130 may include additional modules and/or services for receiving and processing quantitative data. The modules 130 may be implemented using any suitable computer programming language(s) (e.g., Python, JavaScript, C, C++, Rust, C#, Swift, Java, Go, LISP, Ruby, Fortran, etc.). The memories may be non-transitory memories.

The modules 130 may include a machine learning model training module 152, including a plurality of sub-modules. Specifically, the sub-modules may include a cohort selection module 154, a bias correction module 156, a clinical risk module 158 and a survival modeling module 160. In some aspects, more or fewer modules 130 may be included. The modules 130 may be configured to communicate with one another (e.g., via inter-process communication, via a bus, message queue, sockets, etc.).

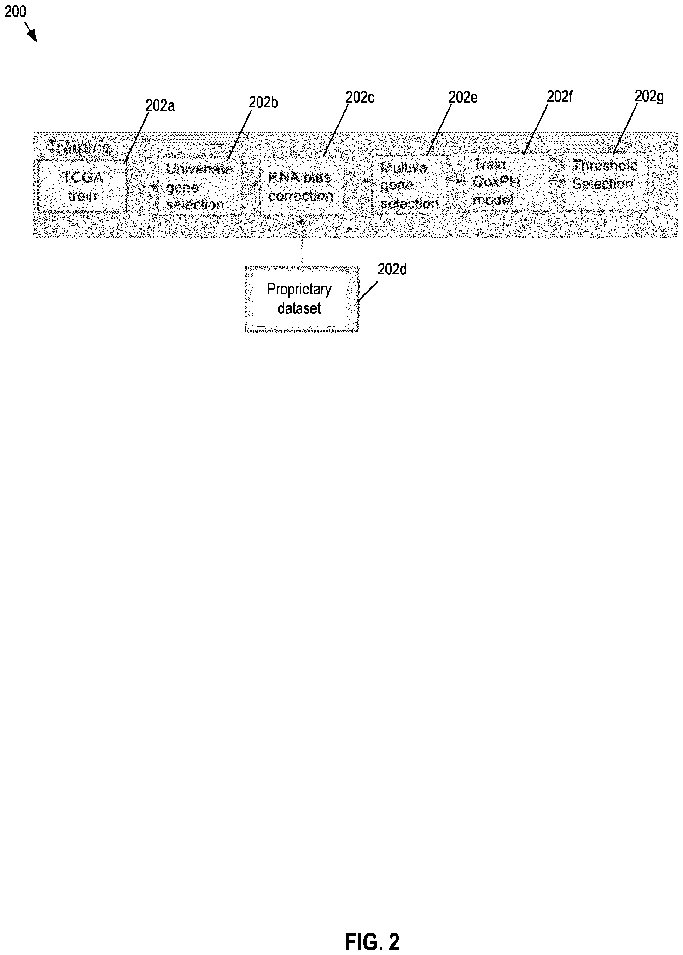

The machine learning model training module 152 may include sets of computer-executable instructions for training one or more machine learning models based on training data. The machine learning model training module 152 may take input data, for example in the form of a dataset, and use it to train a machine learning model. The machine learning model training module 152 may prepare the input data by performing data cleaning, feature engineering, data splitting (into training and validation sets), and handling missing values or outliers. The machine learning model training module 152 may select a machine learning algorithm or model architecture to use for the task at hand. Specifically, the machine learning model training module 152 may include sets of computer-executable instructions for implementing machine learning training architectures such as the cascaded model architecture 200 depicted in FIG. 2. The machine learning model training module 152 may delegate training steps to one or more of the sub-modules 154-160, in some aspects, to perform one or more stages of a training process.

The machine learning model training module 152 may include instructions for performing hyperparameter tuning (e.g., settings or configurations for the model that are not learned from the data but need to be specified before training). The machine learning model training module 152 may use grid search or other techniques for specifying hyperparameters. For example, the machine learning model training module 152 may include instructions to select the following hyperparameters: RNA correction, univariate gene selection, penalizer, L1-ratio, Hcoef, Lcoef, and alpha.

The machine learning model training module 152 may include instructions for training the selected machine learning model on the training data. The training process involves optimizing the model's parameters to make predictions. In some aspects, one or more free/open source software libraries may be used to implement one or more training strategies. Examples of such libraries are: Scikit-learn, Python and Lifelines. For example, the survival modeling module 160 may use CoxPHFitter from the Lifelines library (https://lifelines.readthedocs.io/en/latest/fitters/regression/CoxPHFitter.html) to implement a Cox proportional hazards algorithm, in some aspects. After training, the machine learning model training module 152 may evaluate the trained model's performance using validation data. Validation techniques may include cross-validation as discussed in further detail below.

The machine learning model training module 152 may include instructions for serializing and deserializing stored models. This enables the machine learning model training module 152 to store the trained model as data, and to reload the model and use the trained model for prediction without retraining the model.

The cohort selection module 154 may include instructions for identifying one or more patients, as discussed in further detail below. Specifically, the cohort selection module 154 may include instructions performing univariate gene selection on a patient training dataset, which may include data from a public source (e.g., TCGA) and/or a proprietary source.

The bias correction module 156 may include instructions that remove biases between data sets, as discussed in further detail below.

The clinical risk module 158 may include instructions for determining patient clinical risk, as discussed herein. Clinical risk generally relates to risk that is quantifiable by reference to information available to the clinician without molecular analysis. The clinical risk module 158 may include instructions for determining clinical data from patient records (e.g., via public or proprietary datasets). The clinical risk module 158 may include instructions for retrieving clinical data (e.g., electronic health records) from the database 110, via the API 112, or via another source.

The survival modeling module 160 includes computer-executable instructions for generating survival curves. The instructions may also generate data corresponding to time-to-event predictions using algorithms (e.g., Cox Proportional Hazards) that can use censored data, that are capable of assessing effects of multiple variables on survival time simultaneously. The instructions may include additional or different algorithms, such as Kaplan-Meier estimators that may be used to determine survival probabilities and the effects of different covariates on survival time.

The model operation module 170 may include computer-executable instructions for operating one or more trained machine learning models. For example, the model operation module 170 may have access to next-generation sequencing data via the sequencer 108 and/or the database 110, in some aspects. The model operation module 170 may load one or more model trained by the model training module 152. The model operation module 170 may receive raw next generation sequencing data, preprocess it, apply one or more trained machine learning models, and generate one or more predictions (e.g., a molecular risk prediction, a matched therapy, etc.) corresponding to the next generation sequencing data of the patient.

The model operation module 170 may receive next-generation sequencing data including DNA sequencing data (e.g., whole-genome sequencing, exome sequencing), RNA sequencing data (RNAseq), ChIP sequencing data (ChIP-seq), etc. The model operation module 170 may include instructions for receiving and processing data encoded in multiple different formats (FASTQ, BAM, VCF, etc.).

The model operation module 170 may perform quality control, read alignment, variant calling, and data normalization.

The model operation module 170 may perform feature engineering to transform raw sequencing data into features that can be used by one or more machine learning models trained by the machine learning training module 170.

The model operation module 170 may process the received sequencing data using one or more trained machine models (e.g., a deep learning model, a neural network, a survival model, support vector machine, etc.) to preprocessed sequencing data. The model operation module 170 may generate model performance statistics, such as accuracy, precision, recall, F1-score, or AUC-ROC for classification techniques.

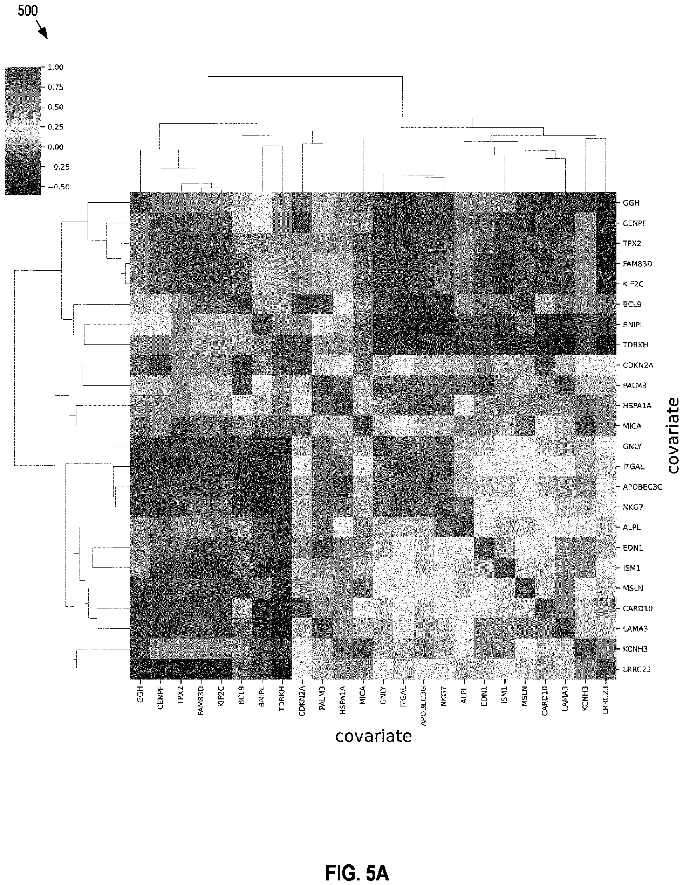

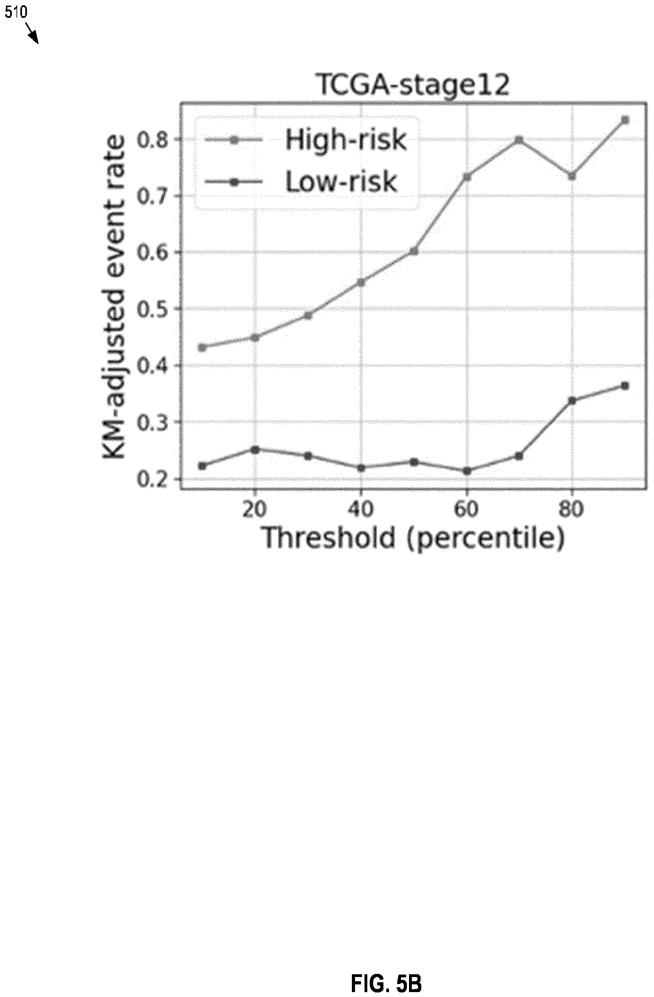

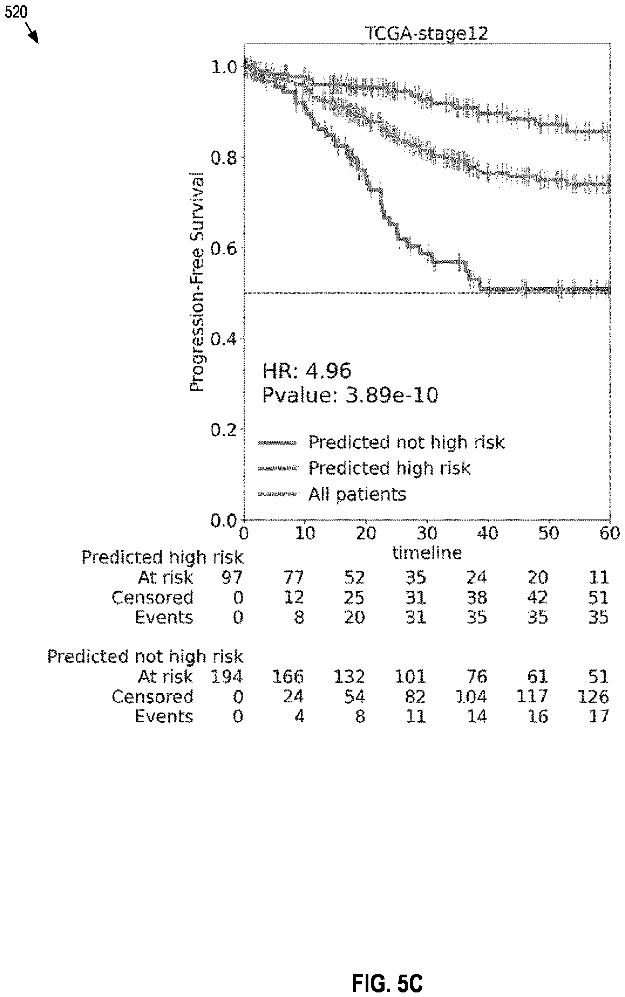

In some aspects, the report generation module 158 may generate reports that include predictions regarding the confidence of classifications of patient data. This data may be plotted, for example in a heatmap, as shown in FIG. 5A. Generally, such a heatmap may be a graphical representation of data where individual values contained in a matrix are colored, and wherein intensity represents higher values of expression for gene covariates, shown on the axes. The squares in a plot may correspond to values from the matrix, and represent a magnitude of the values therein. The instructions may include generating digital visual products to help in understanding. For example, in FIG. 5, the color red represents higher values (higher expression).

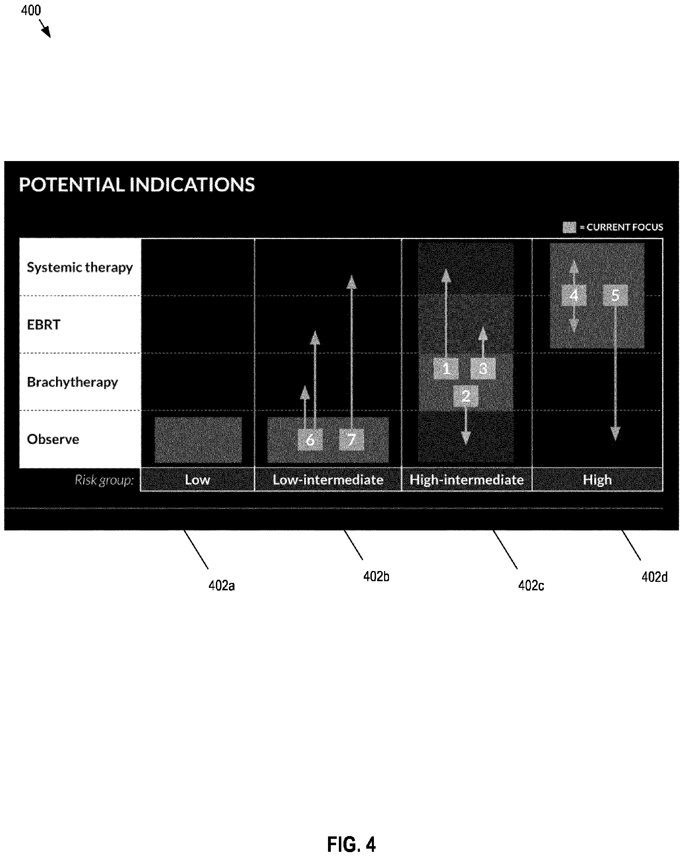

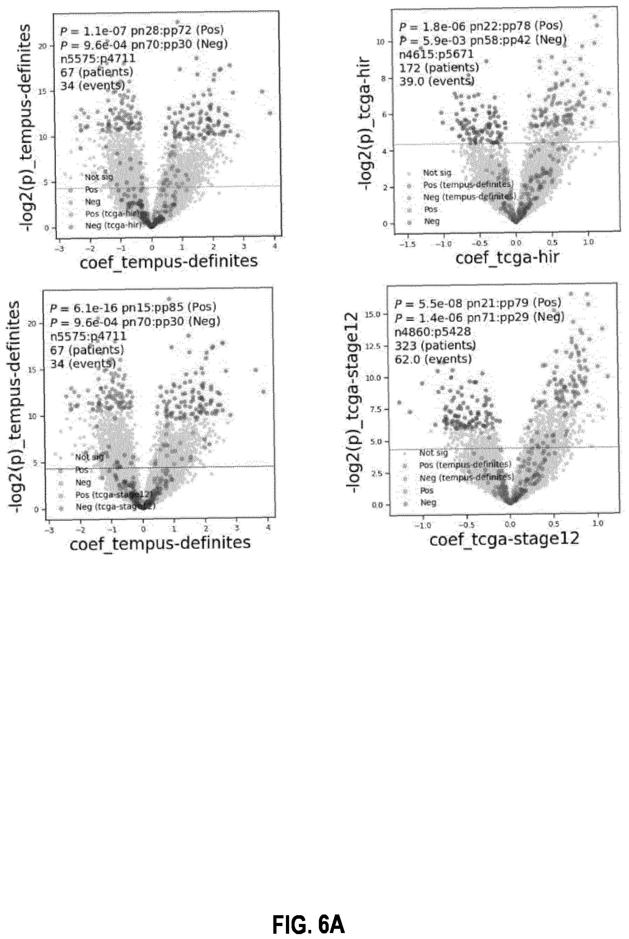

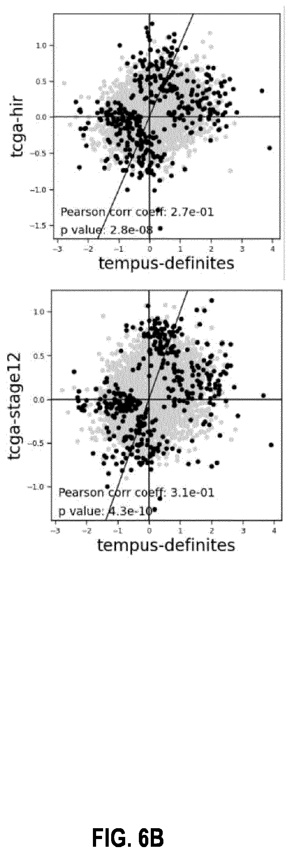

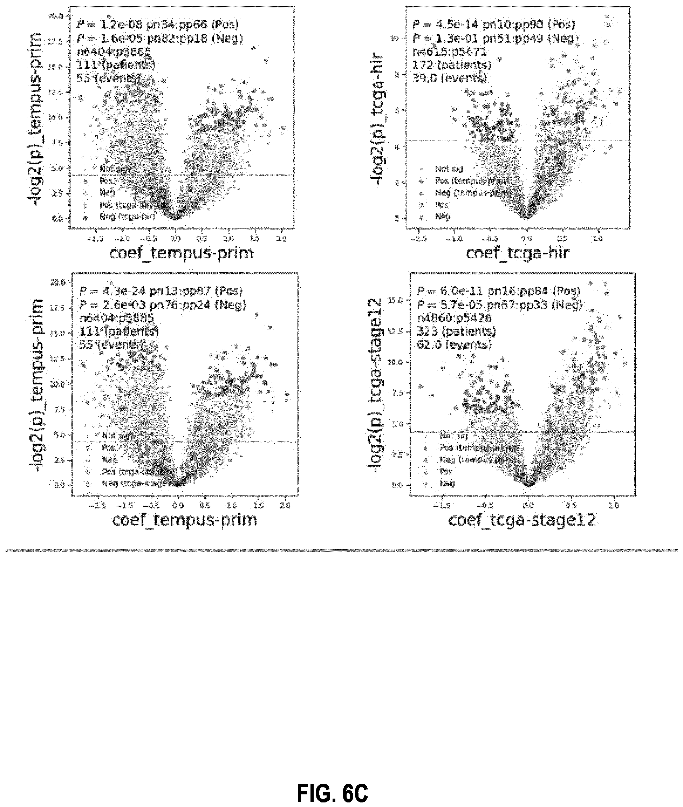

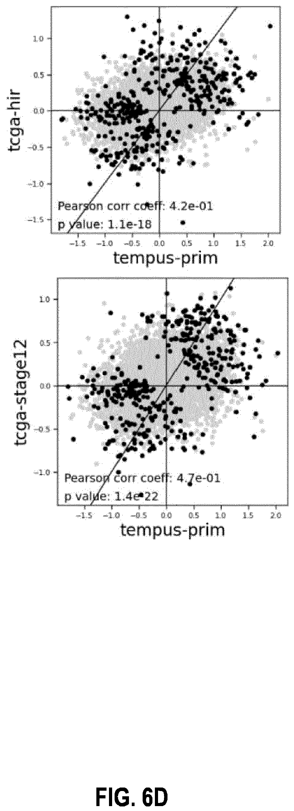

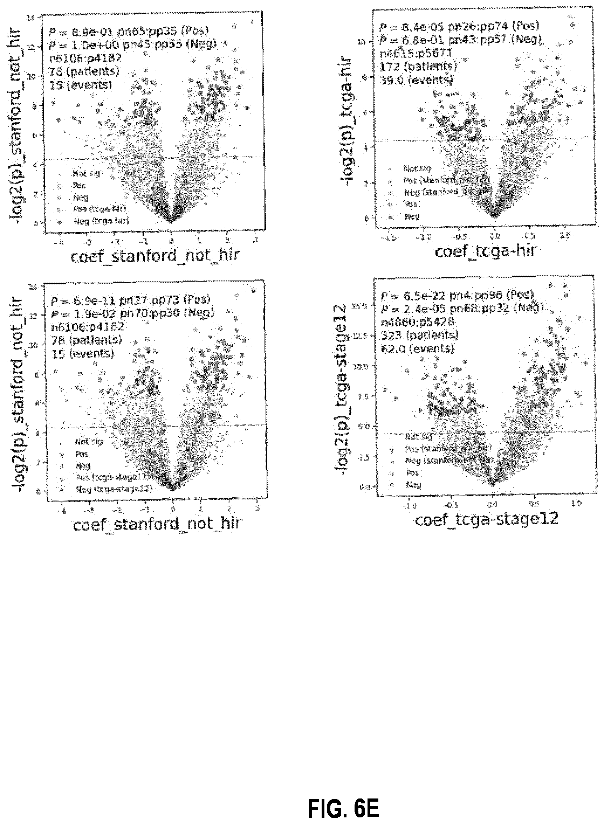

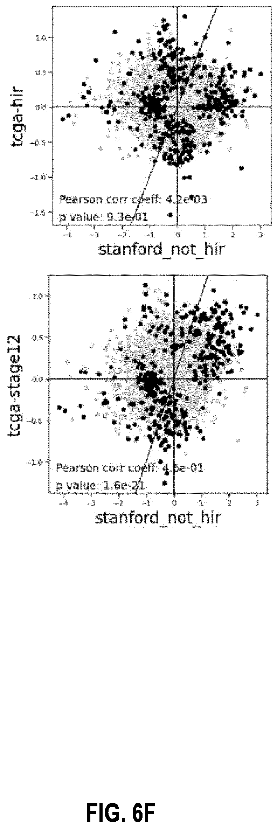

For example, the digital products generated by the report generation module 158 may be shown to clinicians, patients, etc. and may take the form of text documents, digital presentations, word processing documents, etc. For example, the report generation module 158 may generate one or more reports that include a RNA risk score and/or one or more matching therapies of a patient. The reports may include stratified clinical risk and/or stratified molecular (e.g., RNA) risk scores, as shown in FIG. 4, FIG. 5B and FIG. 5C. In some aspects, the report generation module 158 may include instructions for generating comparative/validation visual reports such as the graphs of FIGs. 6A-6F.

The report generation module 158 may include computer-executable instructions for generating machine-readable results. For example, the client computing device 104 may be accessed by a user to view result of generated by the prediction computing device 102. For example, a user may access a mobile device, laptop device, thin client, etc. embodied as the client computing device 104 to view simulation and confidence scoring results, and/or reports, with respect to a sample whose values were processed by the prediction computing device 102. Information from the prediction computing device 102 may be transmitted via the network 106 (e.g., for display via the viewer application 180).

The electronic network 106 may communicatively couple the elements of the environment 100. The network 106 may include public network(s) such as the Internet, a private network such as a research institution or corporation private network, and/or any combination thereof. The network 106 may include a local area network (LAN), a wide area network (WAN), a cellular network, a satellite network, and/or other network infrastructure, whether wireless or wired.

In some aspects, the network 106 may be communicatively coupled to and/or part of a cloud-based platform (e.g., a cloud computing infrastructure). The network 106 may utilize communications protocols, including packet-based and/or datagram-based protocols such as Internet protocol, transmission control protocol, user datagram protocol, and/or other types of protocols. The network 106 may include one or more devices that facilitate network communications and/or form a hardware basis for the networks, such as one or more switches, one or more routers, one or more gateways, one or more access points (such as a wireless access point), one or more firewalls, one or more base stations, one or more repeaters, one or more backbone devices, etc.

The sequencer system 108 may include a next generation sequencer, such as a Tempus xT RNA sequencing whole exome capture transcriptome assay.

The electronic database 110 may include one or more suitable electronic databases for storing and retrieving data, such as relational database (e.g., MySQL databases, Oracle databases, Microsoft SQL Server databases, PostgreSQL databases, etc.). The electronic database 110 may be a NoSQL database, such as a key-value store, a graph database, a document store, etc. The electronic database 110 may be an object-oriented database, a hierarchical database, a spatial database, a time-series database, an in-memory database, etc. In some aspects, some or all of the electronic database 110 may be distributed.

In operation, one or more sequencer runs may be performed using the sequencer 108, either by the company operating/ controlling the environment 100 or by another party. The results of the sequencer may be received by the molecular risk status prediction computing device 102 as sequencer data. The molecular risk status prediction computing device 102 may preprocess the sequencer data, optionally storing some or all of it in the electronic database 110. of FIG. 1. The molecular risk status prediction computing device 102 may load one or more trained models and provide the sequencer data as input to the one or more trained models, and receive predictions as to molecular risk from the one or more trained models. The molecular risk status prediction computing device 102 may provide the sequencer data (e.g., RNASeq data) to the trained machine learning model as a molecular signature. The molecular risk status prediction computing device 102may generate a prediction as to the samples corresponding to the RNASeq. For example, the prediction may correspond to the patient's molecular risk profile (e.g., RNA risk). The molecular risk status prediction computing device 102 may communicate the outcome of the modeling to the patient and/or to a clinician. The molecular risk status prediction computing device 102 may access the patient's current clinical risk profile, to determine whether the model-predicted RNA risk is the same. The molecular risk status prediction computing device 102 may generate one or more matchings of treatment strategies (e.g., as discussed below with respect to FIG. 4) to the patient's current molecular risk and/or clinical risk profiles.

In some aspects, the database 110 may include additional clinical and molecular patient data. For example, the database 110 may include qualitative insights into patient history, symptoms, and clinical reasoning behind treatment decisions, offering a narrative context to the quantitative data also stored within the database. The database 110 may include lab results and test results, ranging from basic blood tests, imaging, or analyses of images, to more complex genetic screenings. The database 110 may store detailed diagnoses, and DNA and RNA sequencing data. In some aspects, the database 110 may include methylation assay results, or other information related to epigenetic modifications or disease etiology. In some aspects, the database 110 may include therapy response data or other tracking information related to how patients respond to various treatments. The database 110 may also incorporate results from the methods described in the application, such as risk profiles generated through the application's trained multi-stage machine learning architecture. By integrating these results, the database enhances the utility of molecular data in clinical decision-making, enabling healthcare providers to identify patients who may benefit from targeted therapies based on their risk profile. This capability represents a significant advancement in the field of oncology, where risk profile is an important factor in determining the most appropriate treatment for cancer patients.

By the time that the molecular risk status prediction computing device 102 uses the trained models in this way, the trained models have already been trained using a training data set as described herein, wherein one or more sub-models that are part of the model architecture (e.g., the model architecture 200) are individually trained to perform tasks such as univariate gene selection, RNA bias correction, multivariate gene selection, survival model training and threshold selection (e.g., using proprietary and/or public training datasets).

Predictions of the molecular risk status prediction computing device 102 may be stored, for example in the memory 124 or the database 110. These results may be provided directly to other elements of the environment 100, for example, via the network 100. These results may also be processed further, for example, to identify/notify patients/clinicians and/or to generate digital reports by the report generation module 158.

## Exemplary Computer-Implemented Machine Learning Models

FIG. 2 depicts an exemplary computer-implemented machine learning model training method 200, according to some aspects. The method 400 may be implemented using the environment 100 of FIG. 1. In some aspects, the method 200 includes identifying a patient training dataset by performing cohort selection (block 202a), as discussed below. The patient training dataset may be received from a database, such as the database 110 of FIG. 1, and may include public data (e.g., data from The Cancer Genome Atlas (TCGA)) and/or proprietary datasets.

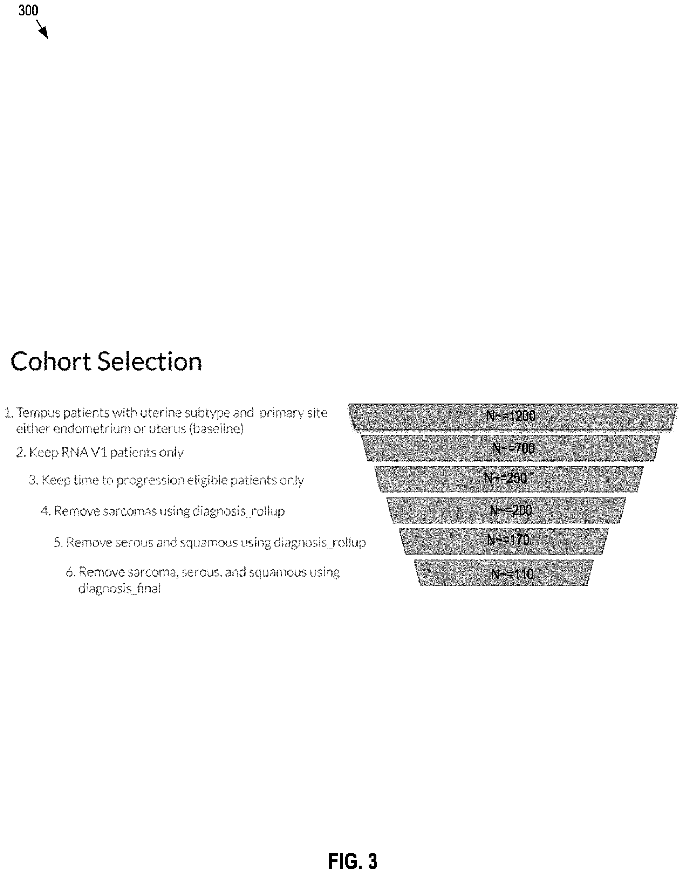

FIG. 3 depicts a method 300 that the method may use at block 202a for performing the cohort selection in cases of endometrioid endometrial cancer, as discussed in further detail below. The method 200 may include performing univariate gene selection/ biomarker identification (block 202b).

The method 200 may include performing dataset correction for technical RNA biases (e.g., domain adaptation). (block 202c) with regard to the reference training dataset (e.g., a matching Tempus RNA dataset) (block 202d). For example, the method 200 may include correcting technical RNA biases between TCGA and Tempus assays using a reference cohort of Tempus primaries (e.g., n=111) and selecting the relevant bias correction hyperparameters in a 3-fold cross-validation experiment. The corrected cohort may be referred to as the Tempus-adapted TCGA cohort (TATC). In some aspects, the SpinAdapt technology may be used to perform correction of RNA data between labs. This may make external datasets (e.g., TCGA) appear as if they were sequenced internally, and is extendable to other data modalities such as DNA and electronic medical records. Using SpinAdapt may remove unnecessary bioinformatics burdens, and allows application of existing molecular predictors on external data without retraining. SpinAdapt is a privacy-preserving technology, which is advantageous. SpinAdapt is the only known algorithm that allows application to new prospective data, without requiring sharing of actual patient data, but rather summary statistics. For example, the techniques described in any of the following publications may be used for RNA correction in the present techniques: U.S. Patent Application No. 17/405,025, filed August 8, 2021, entitled "Systems and Methods for Homogenization of Disparate Datasets"; U.S. Patent Application No. 17/548,118, filed December 10, 2021, entitled "Systems and Methods for Homogenization of Disparate Datasets"; and U.S. Patent Application No. 17/548,084, filed December 10, 2021, entitled "Systems and Methods for Homogenization of Disparate Datasets". Each of the foregoing publications are hereby incorporated by reference in their respective entireties, for all purposes.

The method 200 may include performing multivariate gene selection / gene signature (block 202e). Model training at blocks 202b-202e is further described below, with respect to FIG. 5A, FIG. 5B and FIG. 5C. The method 200 may include selecting a gene signature on the TATC cohort, using a multivariate cox proportional-hazards (CoxPH) model with elastic net penalization. The relevant hyperparameters may be selected in a 3-fold cross-validation experiment. For gene signature characterization, the selection procedure may be repeated on a number (e.g., 1000) of bootstraps of TATC, and the method 200 may select genes enriched in hallmark gene sets including estrogen, proliferation, and invasion gene groups.

The method 200 may include training a survival model (e.g., a Cox proportional hazards (CoxPH) model) (block 202f). The method 200 may include selecting hyperparameters for blocks 202b-202e, using hyperparameter optimization, as discussed herein (block 202g). The method 200 may include training the survival model at block 202f by performing blocks 202b-202e, using the patient training dataset selected at block 202a, the gene selection hyperparameters and survival model hyperparameters determined at block 202g. The method 200 may include selecting a decision threshold on output of the trained survival model (e.g., 70th percentile of the log partial hazard score as evaluated on the corrected training dataset) to identify a molecular high-risk patient population (i.e., a population with an negative, or unfavorable, prognosis).

Specifically, a binary decision threshold may be set at the 60th, 65th, 70th, 75th, or 80th percentile of the log partial-hazard score on the training data. The method 200 may select a cutoff percentile based on model performance in the training cohort. In some aspects, this thresholded model may categorize patients as being molecular risk-high(molecular risk score > 0.255) or molecular risk low (MR score $\leq$ 0.255).

The method 200 may train the survival model (e.g., CoxPH) on the selected genes to predict molecular risk. The molecular risk may be characterized by the log partial-hazard score, and thus the molecular risk is linearly proportional to the expression of the selected genes. The selected gene signature may include: ALPL, APOBEC3G, BCL9, BNIPL, CARD10, CDKN2A, CENPF, EDN1, FAM83D, GGH, GNLY, HSPA1A, ISM1, ITGAL, KCNH3, KIF2C, LAMA3, LRRC23, MICA, MSLN, NKG7, PALM3, TDRKH, TPX2. In some aspects, the selected gene signature my include one or more of the foregoing genes, two or more of the foregoing genes, three or more of the foregoing genes, four or more of the foregoing genes, five or more of the foregoing genes, or more of the foregoing genes.

To evaluate the gene signature stability, the method 200 may repeat the training on multiple bootstraps of the training dataset, with the pre-selected hyperparameters. The idea of repeating through bootstrapping is to analyze various bootstraps of the training population, and compile a list of genes that are consistently selected across bootstraps, ensuring that the gene signature is reproducible across bootstraps. In some aspects, the method 200 may run the bootstrapping experiment 250 times, with the chosen hyperparameters, and the method 200 may append to a list the top 100 genes that were selected most frequently across the 250 bootstraps.

The method 200 may analyze how many of the genes in the gene signature were among the top 100 bootstrap genes (i.e., signature stability). In empirical testing, 80% of the genes from the signature (e.g., of 24 genes) are among the top ~35 genes from the bootstrap list, and almost all of the signature genes are among the top 100 bootstrap genes.

The method 200 may also perform gene set enrichment analysis. For example, the method 200 may performed gene set enrichment analysis on the list of top 100 bootstrap genes, to characterize the type of genes that get selected by the method 200, with the pre-selected set of hyperparameters. The following hallmark gene sets have statistically significant overlap with the gene list (adjusted p-value<0.05): G2-M Checkpoint, Estrogen Response Early, Estrogen Response Late, E2F Targets, Epithelial Mesenchymal Transition, Angiogenesis, MEL18 DN.V1 UP, P53 DN.V1 DN.

The method 200 may train the survival model on the selected gene signature (e.g., 24 genes). The patient's molecular/RNA risk score (low molecular risk or high molecular risk) may be defined as the log-partial hazard output of the survival model. The partial hazard function may be the time-invariant scaling factor in the hazard function, which captures the contribution of covariates (e.g., genes) to the hazard rate. For every $\log_e(k)$ units change in the log-partial hazard score, the hazard rate may be scaled by a factor of k. For example, the hazard rate may doubles if the log-partial hazard molecular risk score increases by $\log_e(2)$ units.

**Exemplary Computer-Implemented Cohort Selection Method Aspects**

FIG. 3 depicts an optional exemplary cohort selection method 300, before the pipeline. In some examples, a universally good machine learning model, that performs well for all target populations, does not exist. Instead, each machine learning model generally performs best for a certain target population. The present techniques may define a target population. Thus, the present techniques may include a cohort selection module that cuts, or stratifies, patient population training data such that the model learns best with respect to the target population. This enables the present techniques to train on large data sets that are aligned with the target population. For example, the cohort selection module may remove histologies evidencing certain attributes (e.g., carcinomas, sarcomas, mixed cells, adenocarcinomas, etc.) from training data through a search process (e.g., via looping). In some aspects, the cohort selection module may use a greedy algorithm strategy to exclude data using one histology attribute after another to determine whether doing so has an impact on matching the training data set with the target population.

For some tasks, the greedy algorithm strategy may result in a statistically significant improvement in match with the target population. For example, empirical testing using the greedy algorithm demonstrated that for training an a cancer prognosticator (e.g., an endometrioid endometrial cancer prognosticator), removing sarcomas from the training data set resulted in the training data aligning much better with the target population. Effectively, the greedy algorithm excludes patients from the training data set who have conditions (e.g., sarcomas) that are not seen in the target population.

As discussed, the present techniques may include training an RNA-seq based gene expression profiler machine learning model to prognostically label patients as high RNA risk or low RNA risk, where high RNA risk patients are more likely to have an event (for example, a progression event), and low RNA risk patients are less likely to have an event. This gene expression profile machine learning model may be validated using Tempus data, in some aspects, and trained using public data (e.g., TCGA data). The present techniques may include advantageous improvements over conventional techniques, in particular, by enabling the use of censored patient data for training. For example, the survival modeling module 158 may include instructions for training a survival model (e.g., Cox Proportional Hazards (CoxPH) model) to model survival for censored patients and uncensored patients, enabling training on larger numbers.

Specifically, when trying to generate predictions to stratify patients as high RNA risk or low RNA risk, an event of interest may be distant recurrence (cancer recurring in another part of the body, different from where it was initially detected). For example, the survival modeling module 156 may process a plurality of patient data at a five-year follow up point in time to determine whether the associated patients had a distant recurrence during the previous five year period. Of course, this time period may be set to a different value, or determined dynamically during the study. In any event, this type of modeling can be difficult to achieve using a classifier due to several complications.

A first complication is lack of follow-up. For example, some patients may not have a full five years of follow-up, and their records may be associated with partial data (e.g., six months, two years, three years or less). Although the records of such patients may be incomplete, the data may still reflect a lack (or presence) of a distant recurrence. Conventional classification models are unable to use such patients' training data, because they only classify patients who either did or did not have a distant recurrence within a fixed period common to all patients.

On the other hand, the present techniques are able to capture such partial data of censored patients, and use it for model training (e.g., using a survival model). Thus, the present techniques advantageously enable larger numbers of training data to be used, including data of censored and uncensored patients, enabling training on larger numbers of patient data, and thus improving model robustness.

The clinical risk module 154 may include a set of computer-executable instructions for determining a patient's clinical risk group. Herein, clinical risk is distinct from RNA risk. Specifically, clinical risk refers to a risk group to which the patient is assigned by a clinician, as shown in FIG. 4). In contrast, the present techniques may also generate RNA risk annotations, classifications and/or scores with respect to a patient, which represent a more granular risk based on processing the patient's RNA information using a trained model, for example. The RNA risk annotations are distinct from the clinical risk categories of FIG. 4. For example, a patient may be in the high clinical risk group 402d, but have a computed RNA risk annotation of low risk. It is thus contemplated that in many cases, clinical risk and RNA risk annotations may not agree - and may be at odds.

The clinical risk module 154 may determine a patient's clinical risk group(s) by reference to clinical factors (e.g., age, stage, myometrial invasion status, lymphovascular space invasion (LVSI) status, and/or other clinical factors). The clinical risk module 154 may categorize the patient according to the predefined criteria. For example, the clinical risk module 154 may include instructions for identifying a patient who has a high-intermediate risk of a given cancer (e.g., endometrioid endometrial cancer). For example, the clinical risk module 154 may include a set of rules that determine a patient's membership in a high-intermediate risk population based upon the patient's values regarding one or more of the clinical factors. It should be appreciated that the clinical risk module 154 may also categorize respective patients into other clinical groups, cohorts and populations based upon the patients' clinical factors. For example, another patient cohort (irrespective of risk) is early stage patients.

### Exemplary Computer-Implemented High-Intermediate Risk Cohort Selection Aspects

As discussed, in some aspects, the present techniques may seek to stratify patient cancer risk by targeting a high-intermediate clinical risk cohort of early-stage endometrioid endometrial cancer patients. However, this cohort may have multiple, complex definitions. For example, during the cohort and model development process, the present techniques may include instructions that implement a patient cohort selection algorithm.

For example, in some aspects, the algorithm implemented by the clinical risk module 154 may require a patient to have an endometrioid histology, and to be at a particular stage (e.g., stage I or II). Stage II patients may or may not be of high-intermediate risk, thus, the algorithm may apply published criteria, in some cases.

For example, the clinical risk module 154 may include instructions encoding rules and criteria set forth in Henry M Keys et al., "A phase III trial of surgery with or without adjunctive external pelvic radiation therapy in intermediate risk endometrioid endometrial adenocarcinoma: a Gynecologic Oncology Group study," Gynecologic Oncology, Volume 92, Issue 3, 2004, Pages 744-751, ISSN 0090-8258, https://doi.org/10.1016/j.ygyno.2003.11.048.

(https://www.sciencedirect.com/science/article/pii/S0090825803008631), incorporated herein by reference in its entirety for all purposes (and hereafter referred to as "GOG") or B. G. Wortman et al. "Ten-year results of the PORTEC-2 trial for high-intermediate risk endometrial carcinoma: improving patient selection for adjuvant therapy," Nature, British Journal of Cancer (2018) 119:1067-1074; https://doi.org/10.1038/s41416-18-0310-8; incorporated by reference herein in its entirety for all purposes (and hereinafter referred to as "PORTEC-2") or Scholten et al., "Postoperative radiotherapy for Stage 1 endometrial carcinoma: Long-term outcome of the randomized PORTEC trial with central pathology review," International Journal of Radiation Oncology*Biology*Physics, Volume 63, Issue 3, 2005, Pages 834-838, ISSN 0360-3016, https://doi.org/10.1016/j.ijrobp.2005.03.007. (https://www.sciencedirect.com/science/article/pii/S0360301605004190) incorporated herein by reference in its entirety for all purposes (and hereinafter referred to as "PORTEC".

In some aspects, the patient cohort selection algorithm implemented by the clinical risk module 153 may further require that patients are stage II or grade 3 with deep invasion.

Table 1, below, summarizes definitions that the clinical risk module 154 may implement in code, and apply to patient data (e.g., patient electronic healthcare records):

**Table 1**

| Source | Definition | Notes |
|---|---|---|
| **GOG** | **Ways to qualify:**<br>• 70+ years old with exactly 1 risk factor | • This is the primary definition listed in UpTo-Date |

(continued)

| Source | Definition | Notes |
|---|---|---|
|  | • 50-69 with exactly 2 risk factors<br>• 18-49 with exactly 3 risk factors<br>**Risk factors:**<br>• Grade 2-3<br>• Outer <u>third</u> myometrial invasion *(it must be outer third, not outer half)*<br>• LVSI+ | (https://www.uptodate .com/contents/adjuva nt-treatment-of-intermediate-risk-endometrial-cancer?search=endo metrial%20cancer%2 0high%20intermediat e%20portec&source= search_result&select edTitle=1~150&usag e_type=default&displ ay_r-ank=1)<br><br>• Clinician interviews indicate this is the most agreed-upon definition in the U.S., but not all agree<br>• UpToDate also considers [grade 3 + deep invasion] to be high risk, but this is not clearly established |
| **PORTEC** | **Must have 2 of these 3 factors:**<br>• >60 years old<br>• >50% myo-metrial inva-sion (proxy: T1b or higher, stage 1b or higher)<br>• Grade 3 | Also listed in UpToDate (https://www.uptodate.com/c ontents/adjuvant-treatment-of-intermediate-risk-endometrial-cancer?search=endometrial %20cancer%20high%20inter mediate %20portec&source= |
|  |  | search_result&selectedTitle= 1~150&usage_type=default& display_rank=1) |

(continued)

| Source | Definition | Notes |
|---|---|---|
| **Example of further nuance: the PORTEC-2 study defined the population like this** | **Ways to qualify:** <br>• FIGO 1988 stage 1C (≥50% myometrial invasion) with age greater than 60 and grade 1 or 2; or <br>• FIGO 1988 stage 1B (<50% myometrial invasion) with age greater than 60 and grade 3; or <br>• FIGO 1988 stage 2A (endocervical glandular involvement, which is stage I in FIGO 2009) with any age, except for grade 3 with deep invasion. | |

FIG. 3 depicts a method 300 of performing cohort selection in endometrial cancer, according to some aspects. The method 300 may include identifying patients with uterine subtype and primary site endometrium or uterus (baseline) (block 301). The method 300 may include excluding any patients that are not RNA V1 (block 302). The method 300 may include identifying (and including) time to progression eligible patients only (block 303). The method 300 may include identifying (and excluding) patients having data relating to sarcoma cancers (block 304). The method 300 may include identifying (and excluding) patients having data relating to serous tissue cancers and squamous tissue cancers (block 305). The method 300 may include identifying (and excluding) patients having data relating to sarcoma cancers, serous tissue cancers and squamous tissue cancers.

The patient cohort selection algorithm represents an advantageous improvement over conventional techniques, which as discussed above, do not define whether patients are high-intermediate, as confirmed by interviews of clinicians and review of clinical trials.

**Exemplary Computer-Implemented Risk Stratification Model Training Aspects**

Using RNA, stratify the patients within this group into high risk RNA patients and low risk RNA patients. Clinicians (e.g., oncologists) may then use this information to treat high-intermediate clinical risk group patients with chemotherapy, even if they are early stage patients.

Step-wise feature selection, signature selection is used. This is a lower-cost computational method, for performing coarser gene selection.

Order of 20,000 genes to 1000 genes. Smaller candidate gene set to run more complex, computationally intensive and possibly more data hungry methods for more granular features selection.

Less precise, less accurate and faster method to perform a first coarser selection, followed by more complex models on a refined candidate set.

This makes the problem computationally feasible and also, enables methods that are more data-hungry to be run on a smaller candidate set.

RNA bias correction may be performed to make two or more datasets having distinct distributions more similar so they may be used together as training data. This bias correction may be performed between two steps. Doing this in the middle enables learning of bias correction between biased RNA data sets to occur using enough data to learn good mappings between the two biased RNA data sets, without having so much data (i.e., so many genes) that the model is overwhelmed, or flooded by a large number of genes such that RNA correction mappings are underfit.

The ordered combination of univariate selection, followed by bias correction, followed by multivariate selection enables good mappings to be learned between biased RNA data sets, without having so many genes that it becomes difficult to learn the biases.

The present techniques may include a model trained using molecular data (e.g., RNA data) that processes a patient's molecular data to generate a high RNA risk annotation that indicates to a clinician or other reviewer that out of a high-intermediate clinical risk group, the patient is more likely to have a distant recurrence, and should be escalated to chemotherapy early. Then, for example, instead of sending a high-intermediate clinical patient home after resection, the patient can be immediately and proactively scheduled for chemotherapy treatment (for example), which may be more likely to lead to a positive patient outcome. The present techniques may identify additional sub-groups, beyond high RNA risk.

For example, FIG. 4 depicts a table 400 of a plurality of clinical risk groups, each of which may be stratified for RNA risk using the present molecular modeling techniques. As discussed herein, the present modeling approaches may be deployed to further stratify risk at each of the clinical risk groups 402. The table 400 includes a low clinical risk group 402a, a low-intermediate clinical risk group 402b, a high-intermediate clinical risk group 402c and a high clinical risk group 402d. The table 400 also includes several clinical treatment strategies 404a-404d, including radiation therapies and chemotherapies. The question presented to the clinician is which of the four therapy strategies to pursue for a given patient. In some aspects, the present techniques may be used to model RNA-based risk of patients in the high-intermediate clinical risk group 402c. For example, when the trained model operated by the model operation module 160 annotates the patient in the high-intermediate clinical risk group 402c as high RNA risk, the patient RNA-based risk stratification computing device 102 may generate an indication that the patient's care should be escalated to the clinical treatment strategy 404a or the clinical treatment strategy 404b. When the present techniques annotate the patient in the high-intermediate clinical risk group 402c as low RNA risk, the patient may be deescalated to observation clinical treatment strategy 404d, thereby avoiding a potentially unnecessary clinical treatment strategy 404c (brachytherapy) from being performed.

Brachytherapy is known to cause a number of common side effects, including fatigue, skin irritation, sexual dysfunction, bowel issues and nausea. Thus, the present techniques include further advantageous improvements over the current state of the art. Specifically, by using trained machine learning models to process patient RNA, the present techniques are able to stratify opaque/homogeneous clinical risk groups into more granular RNA risk annotations, thereby avoiding unnecessary treatment that is likely to cause adverse events or side effects, and/or negatively affect patient outcomes.

This advantage is amplified even further, when considering the high clinical risk group 402d of FIG. 4. This group comprises patients whose clinical risk has been assessed as being high. Thus, on a purely clinical basis, treatment strategies include the treatment strategy 404a (in some examples, systemic therapy, i.e., chemotherapy) and external beam radiation therapy (EBRT). Both of these treatments carry common and significant risks and side effects to patients, including hair loss, fatigue, easy bruising and bleeding, infection, anemia, nausea, sleep disruption, diarrhea and pain. The present techniques are able to stratify some patients in the high clinical risk group 402d with a potentially low RNA risk annotation, which may enable clinicians to recommend avoiding what are otherwise disruptive medical procedures that significantly affect patient outcomes and may be associated with adverse events or side effects. The present techniques thus represent a significant advancement and improvement over conventional cancer prognostic modeling approaches (for example, approaches based on clinical data alone, without considering molecular data).

The present RNA risk modeling techniques are applicable to prognostications for multiple different types of cancer, including endometrioid endometrial cancer, Acute Lymphoblastic Leukemia (ALL), Acute Myeloid Leukemia (AML), Adolescents, Cancer in, Adrenocortical Carcinoma, AIDS-Related Cancers, Kaposi Sarcoma (Soft Tissue Sarcoma), AIDS-Related Lymphoma (Lymphoma), Primary CNS Lymphoma (Lymphoma), Anal Cancer, Appendix Cancer, Astrocytomas, Childhood (Brain Cancer), Atypical Teratoid/Rhabdoid Tumor, Childhood, Central Nervous System (Brain Cancer), Basal Cell Carcinoma of the Skin, Bile Duct Cancer, Bladder Cancer, Bone Cancer (includes Ewing Sarcoma and Osteosarcoma and Malignant Fibrous Histiocytoma), Brain Tumors, Breast Cancer, Bronchial Tumors (Lung Cancer), Burkitt Lymphoma, Carcinoid Tumor (Gastrointestinal), Carcinoma of Unknown Primary, Cardiac (Heart) Tumors, Childhood, Central Nervous System, Atypical Teratoid/Rhabdoid Tumor, Childhood (Brain Cancer), Medulloblastoma and Other CNS Embryonal Tumors, Childhood (Brain Cancer), Germ Cell Tumor, Childhood (Brain Cancer), Primary CNS Lymphoma, Cervical Cancer, Childhood Cancers, Cancers of Childhood, Unusual, Cholangiocarcinoma, Chordoma, Childhood (Bone Cancer), Chronic Lymphocytic Leukemia (CLL), Chronic Myelogenous Leukemia (CML), Chronic Myeloproliferative Neoplasms, Colorectal Cancer, Craniopharyngioma, Childhood (Brain Cancer), Cutaneous T-Cell Lymphoma, Ductal Carcinoma In Situ (DCIS), Childhood (Brain Cancer), Endometrial Cancer (Uterine Cancer), Ependymoma, Childhood (Brain Cancer), Esophageal Cancer, Esthesioneuroblastoma (Head and Neck Cancer), Ewing Sarcoma (Bone Cancer), Extracranial Germ Cell Tumor, Childhood, Extragonadal Germ Cell Tumor, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumor, Gastrointestinal Stromal Tumors (GIST) (Soft Tissue Sarcoma), Germ Cell Tumors, Childhood Central Nervous System Germ Cell Tumors (Brain Cancer), Childhood Extracranial Germ Cell Tumors, Extragonadal Germ Cell Tumors, Ovarian Germ Cell Tumors, Testicular Cancer, Gestational Trophoblastic Disease, Hairy Cell Leukemia, Head and Neck Cancer, Heart Tumors, Childhood, Hepatocellular (Liver) Cancer, Histiocytosis, Langerhans Cell, Hodgkin Lymphoma, Hypopharyngeal Cancer (Head and Neck Cancer), Intraocular Melanoma, Islet Cell Tumors, Pancreatic Neuroendocrine Tumors, Kaposi Sarcoma (Soft Tissue Sarcoma), Kidney (Renal Cell) Cancer, Langerhans Cell Histiocytosis, Laryngeal Cancer (Head and Neck Cancer), Leukemia, Lip and Oral Cavity Cancer (Head and Neck Cancer), Liver Cancer, Lung Cancer (Non-Small Cell, Small Cell, Pleuropulmonary Blastoma, and Tracheobronchial Tumor), Lymphoma, Male Breast Cancer, Malignant Fibrous Histiocytoma of Bone and Osteosarcoma, Melanoma, Melanoma, Intraocular (Eye), Merkel Cell Carcinoma (Skin Cancer), Mesothelioma, Malignant, Metastatic Cancer, Metastatic Squamous Neck Cancer with Occult Primary (Head and Neck Cancer), Midline Tract Carcinoma With NUT Gene Changes, Mouth Cancer (Head and Neck Cancer), Multiple Endocrine Neoplasia Syndromes, Multiple Myeloma/Plasma Cell Neoplasms, Mycosis Fungoides (Lymphoma), Myelodysplastic Syndromes, Myelodysplastic/Myeloproliferative Neoplasms, Myelogenous Leukemia, Chronic (CML), Myeloid Leukemia, Acute (AML), Myeloproliferative Neoplasms, Chronic, Nasal Cavity and Paranasal Sinus Cancer (Head and Neck Cancer), Nasopharyngeal Cancer (Head and Neck Cancer), Neuroblastoma, Non-Hodgkin Lymphoma, Non-Small Cell Lung Cancer, Oral Cancer, Lip and Oral Cavity Cancer and Oropharyngeal Cancer (Head and Neck Cancer), Osteosarcoma and Malignant Fibrous Histiocytoma of Bone, Ovarian Cancer, Pancreatic Cancer, Pancreatic Neuroendocrine Tumors (Islet Cell Tumors), Papillomatosis (Childhood Laryngeal), Paraganglioma, Paranasal Sinus and Nasal Cavity Cancer (Head and Neck Cancer), Parathyroid Cancer, Penile Cancer, Pharyngeal Cancer (Head and Neck Cancer), Pheochromocytoma, Pituitary Tumor, Plasma Cell Neoplasm/Multiple Myeloma, Pleuropulmonary Blastoma (Lung Cancer), Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Rhabdomyosarcoma, Childhood (Soft Tissue Sarcoma), Salivary Gland Cancer (Head and Neck Cancer), Childhood Rhabdomyosarcoma (Soft Tissue Sarcoma), Childhood Vascular Tumors (Soft Tissue Sarcoma), Ewing Sarcoma (Bone Cancer), Kaposi Sarcoma (Soft Tissue Sarcoma), Osteosarcoma (Bone Cancer), Soft Tissue Sarcoma, Uterine Sarcoma, Sézary Syndrome (Lymphoma), Skin Cancer, Small Cell Lung Cancer, Small Intestine Cancer, Soft Tissue Sarcoma, Squamous Cell Carcinoma of the Skin, Squamous Neck Cancer with Occult Primary, Metastatic (Head and Neck Cancer), Stomach (Gastric) Cancer, T-Cell Lymphoma, Lymphoma (Mycosis Fungoides and Sezary Syndrome), Testicular Cancer, Throat Cancer (Head and Neck Cancer), Nasopharyngeal Cancer, Oropharyngeal Cancer, Hypopharyngeal Cancer, Thymoma and Thymic Carcinoma, Thyroid Cancer, Tracheobronchial Tumors (Lung Cancer), Transitional Cell Cancer of the Renal Pelvis and Ureter (Kidney (Renal Cell) Cancer), Ureter and Renal Pelvis, Transitional Cell Cancer (Kidney (Renal Cell) Cancer), Urethral Cancer, Uterine Cancer, Endometrial, Uterine Sarcoma, Vaginal Cancer, Vascular Tumors (Soft Tissue Sarcoma), or Vulvar Cancer.

For example, a breast cancer prognostic model may be trained determine molecular risk, which may be used as discussed herein to determine which patients to escalate. In particular, such a model may escalate newly diagnosed HR+/HER2- patients to endocrine therapy and chemotherapy. Another cancer diagnostic model may be trained and used to predict molecular risk, which may be used to escalate newly diagnosed low stage prostate cancers for some patients. Generally, during treatment of many early stage solid organ cancers, there is a question of whether a patient is best-served by systemic treatment either before or after surgery to mitigate the chance of recurrence and death. An advantageous improvement of the present techniques is basing such decisions of whether to give systemic therapy for early stage patients on prognosis (risk stratification) using clinical, pathologic and/or molecular biomarkers.

In some embodiments, diagnosis may include brain non-glioma (ependymoma, hemangioblastoma, medulloblastoma, meningioma), breast (breast ductal, breast lobular), colon, endometrial (endometrial, endometrial serous, endometrial stromal sarcoma), gastroesophageal (esophageal adenocarcinoma, gastric), gastrointestinal stromal tumor, glioma (Glioma, oligodendroglioma), head and neck adenocarcinoma, hematological (acute lymphoblastic leukemia, acute myeloid leukemia, b cell lymphoma, chronic lymphocytic leukemia, chronic myeloid leukemia, rosai dorfman, t cell lymphoma), hepatobiliary (cholangiocarcinoma, gallbladder, liver), lung adenocarcinoma, melanoma, mesothelioma, neuroendocrine (gastrointestinal neuroendocrine, high grade neuroendocrine lung, low grade neuroendocrine lung, pancreatic neuroendocrine, skin neuroendocrine), ovarian (ovarian clear cell, ovarian granulosa, ovarian serous), pancreas, prostate, renal (renal chromophobe, renal clear cell, renal papillary), sarcoma (chondrosarcoma, chordoma, ewing sarcoma, fibrous sarcoma, leiomyosarcoma, liposarcoma, osteosarcoma, rhabdomyosarcoma, synovial sarcoma, vascular sarcoma), squamous (cervical, esophageal squamous, head and neck squamous, lung squamous, skin squamous/basal), thymic, thyroid, or urothelial.

In some embodiments, diagnosis may include one or more entries of the ICD-10-CM, or the International Classification of Disease. The ICD provides a method of classifying diseases, injuries, and causes of death. The World Health Organization (WHO) publishes the ICDs to standardize the methods of recording and tracking instances of diagnosed disease, including cancer. For example, classifications from any chapter of the ICD or cancers from Chapter 2, C and D codes. C codes may include Neoplasm of Lip, Oral Cavity and Pharynx (COO-C14), Neoplasm of Digestive Organs (C15-C26), Neoplasm of Respiratory System and Intrathoracic Organs (C30-C39), Neoplasm of Mesothelial and Soft Tissue (C45), Neoplasm of Bones, Joints and Articular Cartilage (C40-C41), Neoplasm of Skin (Melanoma, Merkel Cell, and Other Skin Histologies) (C43, C44, C4a), Kaposi Sarcoma (46), Neoplasm of Peripheral Nerves and Autonomic nervous system, Retroperitoneum, Peritoneum, and Soft Tissues (C47, C48, C49), Neoplasm of Breast and Female Genital Organs (C50-C58), Neoplasm of Male Genital Organs (C60-C63), Neoplasm of Urinary Tract (C64-C68), Neoplasms of Eye, Brain and Other Parts of the Central Nervous System (C69-C72), Neoplasm of Thyroid, Other Endocrine Glands, and III-defined Sites (C73-C76), Malignant Neuroendocrine Tumors (C7a._), Secondary Neuroendocrine Tumors (C7B), Neoplasm of other and ill-defined sites (C76-80), Secondary and unspecified malignant neoplasm of lymph nodes (C77), Secondary Cancers of respiratory and digestive organs, other and unspecified sites (C78-80), Malignant Neoplasm without specification of site (C80), Malignant neoplasms of lymphoid, or hematopoietic and related tissue (C81-C96).

**Exemplary Machine Learning Model Training**

Returning to FIG. 2, the method 200 includes performing univariate gene selection/ biomarker identification (block 202b). The method 200 may include performing dataset correction for technical RNA biases (e.g., domain adaptation). (block 202c) with regard to the reference dataset (e.g., a matching Tempus RNA dataset) (block 202d). The method 200 may include performing multivariate gene selection / gene signature (block 202e). For example, training data at block 202 may include datasets with RNASeq data and relevant clinical outcomes. For example, the following training datasets may be used:

    1. TCGA Endometrioid (N=400, clinical endpoint=PFS)

        a. TCGA Stage 1 and 2 (N=300)
        b. TCGA HIRs (N=170)

    2. Tempus Endometrial Primaries (N=110, clinical endpoint=PFS)
    3. Tempus Endometrial HIRs (N=60, clinical endpoint=PFS)
    4. Institutional (N=181, clinical endpoint=Distant Recurrence)

        a. Institutional-not-HIR (N=70)
        b. Institutional HIR (N=100)

            i. GOG (N=50)
            ii. PORTEC (N=80)
            iii. GOG or PORTEC (N=100)

As noted, the method 200 may include hyperparameter optimization at block 202g. For example, during hyperparameter search/optimization, performance may be evaluated on Institutional-not-HIR for hyperparameter selection.

-     Hyperparameters:
-     RNA correction methods: [z-score, spinadapt, mean correction]
-     Reference for RNA correction: [Tempus primaries]
-     Univariate gene selection: [300,400,500]
-     Penalizer: [1e-3,1e-2,1e-1,1e0, 1e1]
-     L1-ratio: [1e-2, 1e-1 ,0.2,0.3,0.5,1]
-     Hcoef: [0.05, 0.1, 0.25]
-     Lcoef: [0.05, 0.1, 0.25]
-     Alpha: [0.01,0.05,0.1,0.15,0.2,0.25,0.3,0.5]

Hyperparameter search: Performance metrics

Train on TCGA Stage 1 and 2 patients with RNASeq corrected to Tempus RNA V2 endometrial cancer primaries. Optimize hyperparameters on Institutional-not-HIR dataset. Performance metrics are show in Table 2, below.

**Table 2**

| Optimization metric | l1 ratio | alpha | lcoef | hcoef | univ genes + | univ genes - | metric value | genes |
|---|---|---|---|---|---|---|---|---|
| -log10 P-val (top 10%) | 0.1 | 0.3 | -0.1 | 0.1 | 500 | 500 | 7.71 | 57 |
| -log10 P-val (top 20%) | 0.2 | 0.2 | -0.1 | 0.1 | 500 | 500 | 3.89 | 35 |
| -log10 P-val (top 30%) | 0.2 | 0.2 | -0.1 | 0.1 | 500 | 500 | 3.95 | 35 |
| -log10 P-val (top 40%) | 0.2 | 0.15 | -0.1 | 0.25 | 500 | 500 | 3.07 | 45 |
| -log10 P-val (top 20%) transfer threshold | 0.1 | 0.1 | -0.1 | 0.25 | 500 | 500 | 6.64 | 64 |
| -log10 P-val (top 30%) | 0.2 | 0.2 | -0.1 | 0.25 | 500 | 500 | 3.82 | 45 |
| transfer threshold | | | | | | | | |
| -log10 P-val (top 40%) transfer threshold | 0.2 | 0.2 | -0.1 | 0.25 | 500 | 500 | 3.77 | 45 |
| C-index | 0.2 | 0.2 | -0.1 | 0.1 | 500 | 500 | 0.80 | 35 |
| KM Event Rate-top 20% | 0.2 | 0.2 | -0.1 | 0.1 | 500 | 500 | 0.56 | 35 |
| KM Event Rate-top 40% | 0.2 | 0.2 | -0.1 | 0.1 | 500 | 500 | 0.41 | 35 |

Once hyperparameters are selected, the method 200 may train on all TCGA-stage12 patients (n=323) using the method 200.

At block 202a, the method 200 may perform univariate gene selection to lower the number of admissible genes. The method 200 may regress expression of each gene on survival data across all patients (e.g., using univariate cox model). The method 200 may set tunable parameters, by choosing K most important genes (e.g., K ~ 500). In some aspects, expression of each gene may be regressed on survival data across all TCGA patients using a univariate cox model, to select a set of candidate genes (e.g., 1000 genes).

For example, at block 202f, the method 200 may perform multivariate gene selection using a multivariate (e.g., CoxPH) survival model with Elastic Net penalty. Tunable parameters may include regularization term, L1 and L2 penalization weights:

$$\arg\max_{\beta} \quad \log \mathrm{PL}(\beta) - \alpha \left( r \sum_{j=1}^{p} |\beta_j| + \frac{1-r}{2} \sum_{j=1}^{p} \beta_j^2 \right)$$

These tunable parameters determine the number of genes, and they may be selected (e.g., in a 3-fold CV experiment). The method 200 may initialize model training with the selected hyperparameters to select our genes and train the Cox PH model at block 202f. The method 200 may perform threshold selection at block 202g, by selecting the threshold to be 70th percentile of log-partial hazard scores on the TCGA-stage12 dataset. FIG. 5A depicts heatmap 510 showing the clustering of a final gene set as determined by the method 200 of FIG. 2.

The method may evaluate the trained survival model (e.g., Cox PH) on the TCGA-stage12 training dataset via calibration plot for the low-risk arm and the high-risk arm. The two arms may be selected based on a single threshold determined on the log-partial hazard score of the TCGA-stage12 patients. FIG. 5B depicts a plot 510 of Kaplan-Meier (KM)-adjusted event risk rates, wherein the x-axis corresponds to the percentile on the TCGA-stage12 log-partial hazard scores for the decision threshold.

Any point on the high-risk curve gives 4-year KM-adjusted risk for patients with log-partial hazard score above the reported threshold on x-axis, with percentiles calculated on TCGA-stage12. Any point on the low-risk curve gives 4-year KM-adjusted risk for patients with log-partial hazard score below the reported threshold on x-axis. Of course, the method 200 may compute the risk curves for any suitable time scale, provided sufficient data exists.

FIG. 5B demonstrates the practical applications of the present techniques. First, it shows that the method 200 enables patients to be further stratified, based on their risk levels. Patients with higher log-partial hazard scores may be at a higher risk of the event (e.g., cancer recurrence) within the period of time. FIG. 5B also demonstrates that the different thresholds of the log-partial hazard score can be used to predict outcomes. By observing where the risk significantly changes can help in determining the most appropriate cut-off points for clinical decision-making. FIG. 5B also enables clinicians to compare risk across stages of cancer, to make better treatment decisions and to identify higher risk patients.

FIG. 5C depicts a survival plot 520 for TCGA-stage12 patients, with decision threshold set at 70th percentile of log-partial hazard scores on the TCGA-stage12 dataset.

The invention can be used to leverage publicly available datasets for training RNA-based prognostic models such that the models are not only less expensive to train but also more reproducible across patient cohorts from heterogeneous sources. These models can be used to predict (i) risk of distant recurrence, (ii) risk of locoregional recurrence, and (iii) risk of cancer progression.

Moreover, these models can be readily evaluated on in-house sequenced datasets after standard normalization procedures (VST normalization or log-transformed transcripts per million).

As noted, there are unmet needs in understanding risk to cancer patients, especially high-intermediate risk patients. The present molecular classification and gene profiling techniques are a direct response to those unmet needs, and may be used to predict distant recurrence risk in early-stage endometrioid endometrial cancer with a focus on high intermediate-risk patients. The RNA-seq-based gene expression profiler of the present techniques may be trained using TCGA data, resulting in a gene signature (e.g., a 24-gene signature) that classifies (e.g., labels, profiles or annotates) endometrioid endometrial cancer patients' respective molecular risk (e.g., RNA-based risk prediction) as either high or low. This profiling may be used to further stratify opaque/homogeneous patient groups.

The present genetic expression profiler machine learning techniques may then be tested on a de-identified cohort of endometrioid endometrial cancer patients (e.g., N=~1000) from Tempus to test associations with known pathologic or molecular prognostic features.

Empirical testing has shown that the present genetic expression profiler machine learning techniques show significant enrichment of high molecular risk in G3 versus G1/2 histology (p-value < 5e-8). A high correlation was found between the molecular risk score and copy number alteration score (t-test p-value < 1e-5). Next, a clinical evaluation was performed in an early-stage endometrioid endometrial cancer case-control cohort of patients with documented recurrence or no recurrence event at four years (N=109), from Institutional. In the entire cohort, the high molecular risk group had a significantly higher rate of distant recurrence in comparison to the low molecular risk group (HR=4.8, N=109). Next, a subgroup analysis in the clinically important high-intermediate clinical risk group of patients was performed. In this subgroup, the high molecular risk group had a significantly higher rate of distant recurrence in comparison to the low molecular risk group (HR=8.0, N=56). Lastly, given the significance of genomic biomarkers in the evolution of endometrioid endometrial cancer FIGO staging, outcomes were stratified by the established TCGA subtypes as a reference standard and a subgroup analysis performed in patients classified as having no specific molecular profile (NSMP). Among patients who were NSMP, the high molecular risk molecular group showed a significantly higher rate of distant recurrence in comparison to the low molecular risk group (HR=7.92, N=67). These evaluation studies demonstrate the performance of the gene expression profiler molecular risk machine learning models in early-stage endometrioid endometrial cancer distant recurrence risk stratification, specifically high-intermediate clinical risk patients, and may be used clinically to inform adjuvant clinical management.

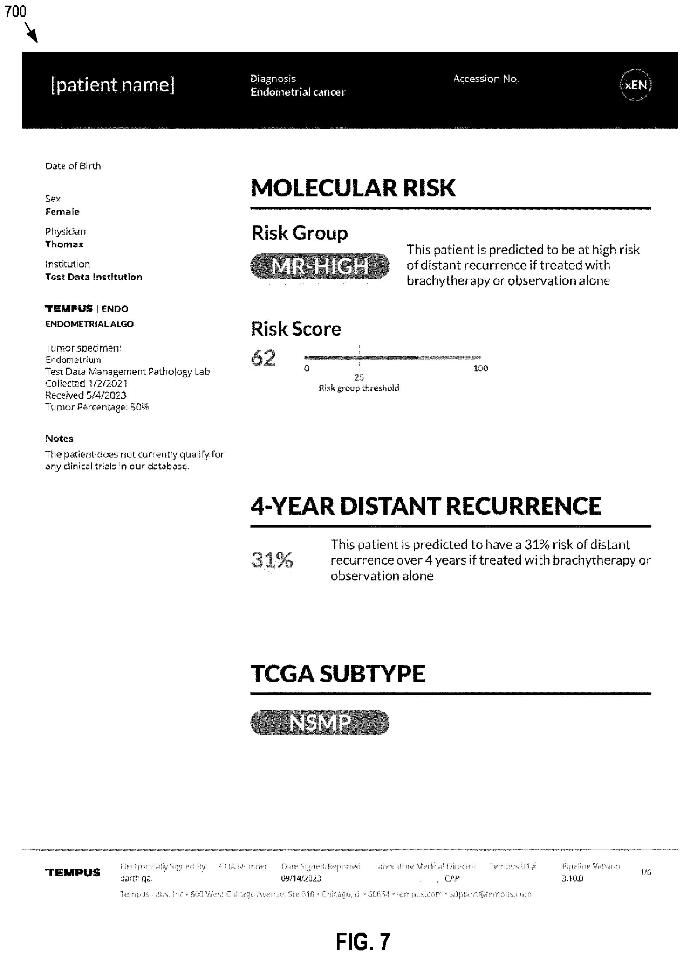

FIG. 7 depicts an exemplary digital report of a patient's molecular risk evaluation and prediction of disease recurrence from a pathology analysis, according to some aspects. The report includes the patient's diagnosis of endometrial cancer and contains several sections detailing the results and their potential clinical implications. The top portion of the report provides a placeholder for the patient's name (redacted), the diagnosis of endometrial cancer, the accession number (redacted), and the following visible details:

- Date of Birth (Not visible)
- Sex (Female)
- Physician (example name Thomas)
- Institution (Not visible)
- Test Data Institution (Collected 1/4/2021, Received 5/4/2023)
- Tumor Specimen (Endometrium)
- Test Data Management Pathology Lab Information (Endometrial Algo)
- Tumor Percentage (50%)

It also states that the patient does not currently qualify for any clinical trials in the database.

Under the "MOLECULAR RISK" section, the report highlights the patient's risk group as "MR-HIGH," with a Risk Score of 62, showing 25 as the risk group threshold on a graphical scale from 0 to 100. The text indicates that "This patient is predicted to be at high risk of distant recurrence if treated with brachytherapy or observation alone."

The "4-YEAR DISTANT RECURRENCE" section indicates that this patient is predicted to have a 31% risk of distant recurrence over 4 years if treated with brachytherapy or observation alone.

The "TCGA SUBTYPE" section shows the subtype as "NSMP" presented as a filled-in bubble format.

The bottom of the report includes an electronic signature by the CLIA number, the Date Signed/Reported (01/14/2023), Laboratory Medical Director, Tempus ID#, and Pipeline Version (3.10.0), as well as the laboratory address (Tempus Labs, Inc. • 600 West Chicago Avenue, Ste 510 • Chicago, IL • 60654 • tempus.com • support@tempus.com).

The report depicted in FIG. 7, which provides a detailed evaluation of a patient's molecular risk and the prediction of disease recurrence for a patient diagnosed with endometrial cancer, can be generated through a series of computational and analytical processes as outlined in the disclosed methods and systems for stratifying patient cancer risk using computational oncology and molecular data. For example, initially, molecular data corresponding to the patient, which may include RNA sequencing data, may be received by the molecular risk status prediction computing device 102. This device is equipped with one or more processors and memories that store computer-executable instructions for processing the molecular data, as discussed. The processing may include the use of a machine learning model that has been trained using a patient training dataset and a reference training dataset. This model is capable of employing techniques such as univariate gene selection, RNA bias correction, and multivariate gene selection to filter and correct its training data. Furthermore, the model may incorporate a survival model, which could be, for example, a Cox Proportional Hazards model. The machine learning model's training may involve several steps, including the selection of a cohort of patients from the patient training dataset, the selection of genes using univariate and multivariate gene selection methods, and the correction of biases in the molecular data of the patients. This process may also involve optimizing hyperparameters to improve the model's accuracy in predicting molecular risk. The training data used for this purpose could come from various sources, including public datasets like The Cancer Genome Atlas (TCGA) and proprietary datasets from institutions (e.g., Tempus AI, Inc.).

Once the model is trained, it processes the received molecular data to determine the patient's molecular data risk. This may include analyzing the patient's molecular data against the trained model to classify the patient's risk as high or low. In the case of the report in FIG. 7, the patient is classified as having a high molecular risk ("MR-HIGH") with a specific risk score indicated on a graphical scale.

Based on the patient's molecular data risk, a matched treatment strategy is generated. This strategy takes into account the patient's molecular risk profile and suggests appropriate treatment options. For the patient in FIG. 7, the report indicates a high risk of distant recurrence if treated with brachytherapy or observation alone, suggesting that alternative treatment strategies may be more appropriate.

The report also includes additional sections such as the "4-YEAR DISTANT RECURRENCE" which provides a quantitative risk assessment of distant recurrence over a four-year period, and the "TCGA SUBTYPE" which classifies the patient's cancer subtype based on molecular data.

The generation of this report may be facilitated by the report generation module 158, which compiles the analysis results, predictions, and recommendations into an understandable format. This module may also include instructions for generating comparative/validation visual reports, enhancing the interpretability of the data for clinicians and patients.

The various embodiments described above can be combined to provide further embodiments. All U.S. patents, U.S. patent application publications, U.S. patent application, foreign patents, foreign patent application and non-patent publications referred to in this specification and/or listed in the Application Data Sheet are incorporated herein by reference, in their respective entireties, for all purposes. Aspects of the embodiments can be modified if necessary to employ concepts of the various patents, applications, and publications to provide yet further embodiments.

These and other changes can be made to the embodiments in light of the above-detailed description. In general, in the following claims, the terms used should not be construed to limit the claims to the specific embodiments disclosed in the specification and the claims but should be construed to include all possible embodiments along with the full scope of equivalents to which such claims are entitled. Accordingly, the claims are not limited by the disclosure.

Aspects of the techniques described in the present disclosure may include any of the following aspects, either alone or in combination:

1. A computer-implemented method for stratifying patient cancer risk using molecular data, comprising: receiving, via one or more processors, molecular data corresponding to a patient; processing, via one or more processors, the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and generating a matched treatment strategy corresponding to the patient based upon the patient's molecular data risk.

2. The computer-implemented method of aspect 1, wherein the survival model is a Cox Proportional Hazards model.

3. The computer-implemented method of any of aspects 1-2, wherein the molecular data corresponding to the patient includes RNA seq data.

4. The computer-implemented method of any of aspects 1-3, wherein the cancer is endometrial cancer, and wherein the machine learning model was trained on a cohort of patient data selected using a greedy algorithm.

5. The computer-implemented method of aspect 4, wherein the greedy algorithm includes: identifying patients with uterine subtype and primary site endometrium or uterus; identifying time to progression eligible patients; identifying

**EP 4 557 297 A1**

patients with sarcoma cancers; identifying patients having serous tissue cancers and squamous tissue cancers; and identifying patients having sarcoma cancers, serous tissue cancers and squamous tissue cancers.

6. The computer-implemented method of any of aspects 1-5, wherein the patient has a pre-existing clinical risk group assignment of at least one of the following: low clinical risk, low-intermediate clinical risk, high-intermediate clinical risk or high clinical risk.

7. The computer-implemented method of aspect 6, wherein generating the treatment strategy corresponding to the patient based upon the patient's molecular data risk includes generating the treatment strategy based upon both of (i) the pre-existing clinical risk group; and (ii) the patient's molecular risk.

8. The computer-implemented method of aspect 7, wherein the pre-existing clinical risk group is high-intermediate, the patient's molecular risk is high, and the treatment strategy is at least one of systemic therapy or external beam radiation therapy.

9. The computer-implemented method of any of aspects 7-8, wherein the pre-existing clinical risk group is high-intermediate, the patient's molecular risk is low, and the matched treatment strategy is observation.

10. The computer-implemented method of any of aspects 7-9, wherein the pre-existing clinical risk group is low-intermediate, the patient's molecular risk is high, and the matched treatment strategy is at least one of brachytherapy, external beam radiation therapy or systemic therapy.

11. The computer-implemented method of any of aspects 7-10, wherein the pre-existing clinical risk group is high, the patient's molecular risk is low, and the matched treatment strategy is observation.

12. The computer-implemented method of any of aspects 7-11, wherein the pre-existing clinical risk group is high, the patient's molecular risk is low, and the matched treatment strategy is observation.

13. The computer-implemented method of any of aspects 7-12, wherein the pre-existing clinical risk group is high, the patient's molecular risk is high, and the matched treatment strategy is at least one of systemic therapy or external beam radiation therapy.

14. The computer-implemented method of any of aspects 1-13, wherein the matched treatment strategy includes at least one of systemic therapy, external beam radiation therapy, brachytherapy or observation.

15. A computer-implemented method for training a machine learning model to stratify patient cancer risk using molecular data, comprising: receiving, via one or more processors: (i) a patient training dataset, wherein the patient training dataset includes respective molecular data of a plurality of patients, and (ii) a reference training dataset including respective molecular data of a plurality of patients; selecting, via one or more processors, a cohort of patients from the patient training dataset; selecting, via one or more processors, a small subset of genes from the patient training dataset using univariate gene selection; generating a corrected reference training dataset by processing the reference training dataset to correct biases in the molecular data of the plurality of patients; selecting, via one or more processors, a smaller subset of genes from the small subset of genes, using multivariate gene selection; training, via one or more processors, a survival model, wherein the training includes determining a set of hyperparameters; and selecting, via one or more processors, a decision threshold to identify a patient population having an RNA risk profile.

16. The computer-implemented method of aspect 15, wherein the patient cancer risk is that of endometrial cancer; and wherein selecting, via the one or more processors, the cohort of patients from the patient training dataset includes applying, via one or more processors, a greedy algorithm that excludes patient data by: identifying patients with uterine subtype and primary site endometrium or uterus; identifying time to progression eligible patients; identifying patients with sarcoma cancers; identifying patients having serous tissue cancers and squamous tissue cancers; and identifying patients having sarcoma cancers, serous tissue cancers and squamous tissue cancers.

17. The computer-implemented method of any of aspects 15-16, wherein the molecular data includes at least some transcriptomic data.

18. The computer-implemented method of any of aspects 15-17, wherein the molecular data includes at least some data generated via RNA seq.

19. The computer-implemented method of any of aspects 15-18, wherein receiving, via the one or more processors the reference training dataset including the respective molecular data of the plurality of patients includes receiving the reference training dataset from a next-generation sequencing platform.

20. A computing system, comprising: one or more processors, and one or more memories having stored thereon computer-executable instructions that, when executed by the one or more processors, cause the computing system to: receive molecular data corresponding to a patient; process the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and generating a matched treatment strategy corresponding to the patient based upon the patient's molecular data risk.

21. The computing system of aspect 20, the one or more memories having instructions stored thereon that, when executed, cause the computing system to perform the functions of any of aspects 2-14.

22. A computing system, comprising: one or more processors, and one or more memories having stored thereon computer-executable instructions that, when executed by the one or more processors, cause the computing system to: receive: (i) a patient training dataset, wherein the patient training dataset includes respective molecular data of a plurality of patients, and (ii) a reference training dataset including respective molecular data of a plurality of patients; select a cohort of patients from the patient training dataset; select a small subset of genes from the patient training dataset using univariate gene selection; generate a corrected reference training dataset by processing the reference training dataset to correct biases in the molecular data of the plurality of patients; select a smaller subset of genes from the small subset of genes, using multivariate gene selection; train a survival model, wherein the training includes determining a set of hyperparameters; and select a decision threshold to identify a patient population having an RNA risk profile.

23. The computing system of aspect 22, the one or more memories having instructions stored thereon that, when executed, cause the computing system to perform the functions of any of aspects 16-19.

22. A computer-readable medium having stored thereon computer-executable instructions that, when executed by one or more processors, cause a computer to: receive molecular data corresponding to a patient; process the molecular data using a machine learning model to determine the patient's molecular data risk, wherein the machine learning model is trained using a patient training dataset and a reference training data set, wherein the machine learning model uses univariate gene selection, RNA bias correction and multivariate gene selection to filter and correct its training data, and wherein the machine learning model includes a survival model; and generating a matched treatment strategy corresponding to the patient based upon the patient's molecular data risk.

23. The computer-readable medium of claim 22, having stored thereon instructions that when executed, cause the computer to perform any of the functions of any of aspects 2-14.

24. A computer-readable medium having stored thereon computer-executable instructions that, when executed by one or more processors, cause a computer to: receive: (i) a patient training dataset, wherein the patient training dataset includes respective molecular data of a plurality of patients, and (ii) a reference training dataset including respective molecular data of a plurality of patients; select a cohort of patients from the patient training dataset; select a small subset of genes from the patient training dataset using univariate gene selection; generate a corrected reference training dataset by processing the reference training dataset to correct biases in the molecular data of the plurality of patients; select a smaller subset of genes from the small subset of genes, using multivariate gene selection; train a survival model, wherein the training includes determining a set of hyperparameters; and select a decision threshold to identify a patient population having an RNA risk profile.

25. The computer-readable medium of claim 22, having stored thereon instructions that when executed, cause the computer to perform any of the functions of any of aspects 16-19.

## ADDITIONAL CONSIDERATIONS

The computer-readable media may include executable computer-readable code stored thereon for programming a computer (e.g., comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device 1300 may represent a CPU-type processing unit, a GPU-type processing unit, a TPU-type processing unit, a field-programmable gate array (FPGA), another class of digital signal processor (DSP), or other hardware logic components that can be driven by a CPU.

A system for performing the methods described herein may include a computing device, and more particularly may be implemented on one or more processing units, for example, Central Processing Units (CPUs), and/or on one or more or Graphical Processing Units (GPUs), including clusters of CPUs and/or GPUs. Features and functions described may be stored on and implemented from one or more non-transitory computer-readable media of the computing device. The computer-readable media may include, for example, an operating system and software modules, or "engines," that implement the methods described herein. Those engines may be stored as sets of non-transitory computer-executable instructions. The computing device may be a distributed computing system, such as an Amazon Web Services, Google Cloud Platform Microsoft Azure, or other public, private and/or hybrid cloud computing solution.

The computing device includes a network interface communicatively coupled to network, for communicating to and/or from a portable personal computer, smart phone, electronic document, tablet, and/or desktop personal computer, or other computing devices. The computing device further includes an I/O interface connected to devices, such as digital displays, user input devices, etc.

The functions of the engines may be implemented across distributed computing devices, etc. connected to one another through a communication link. In other examples, functionality of the system may be distributed across any number of devices, including the portable personal computer, smart phone, electronic document, tablet, and desktop personal computer devices shown. The computing device may be communicatively coupled to the network and another network. The networks may be public networks such as the Internet, a private network such as that of a research institution or a corporation, or any combination thereof. Networks can include, local area network (LAN), wide area network (WAN), cellular, satellite, or other network infrastructure, whether wireless or wired. The networks can utilize communications protocols, including packet-based and/or datagram-based protocols such as Internet protocol (IP), transmission control protocol (TCP), user datagram protocol (UDP), or other types of protocols. Moreover, the networks can include a number of devices that facilitate network communications and/or form a hardware basis for the networks, such as switches, routers, gateways, access points (such as a wireless access point as shown), firewalls, base stations, repeaters, backbone devices, etc.

The computer-readable media may include executable computer-readable code stored thereon for programming a computer (for example, comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device may represent a CPU-type processing unit, a GPU-type processing unit, a field-programmable gate array (FPGA), another class of digital signal processor (DSP), or other hardware logic components that can be driven by a CPU.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components or multiple components.

Additionally, certain aspects are described herein as including logic or a number of routines, subroutines, applications, or instructions. These may constitute either software (e.g., code embodied on a machine-readable medium or in a transmission signal) or hardware. In hardware, the routines, etc., are tangible units capable of performing certain operations and may be configured or arranged in a certain manner. In example aspects, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various aspects, a hardware module may be implemented mechanically or electronically. For example, a hardware module may comprise dedicated circuitry or logic that is permanently configured (e.g., as a special-purpose processor, such as a microcontroller, field programmable gate array (FPGA) or an application-specific integrated circuit (ASIC)) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering aspects in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connects the hardware modules. In aspects in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of the example methods described herein can be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example aspects, comprise processor-implemented modules.

Similarly, the methods or routines described herein may be at least partially processor-implemented. For example, at least some of the operations of a method can be performed by one or more processors or processor-implemented hardware modules. The performance of certain of the operations may be distributed among the one or more processors, not only residing within a single machine, but also deployed across a number of machines. In some example aspects, the processor or processors may be located in a single location (e.g., within a home environment, an office environment or as a server farm), while in other aspects the processors may be distributed across a number of locations.

The performance of certain of the operations may be distributed among the one or more processors, not only residing within a single machine, but also deployed across a number of machines. In some example aspects, the one or more processors or processor-implemented modules may be located in a single geographic location (e.g., within a home environment, an office environment, or a server farm). In other example aspects, the one or more processors or processor-implemented modules may be distributed across a number of geographic locations.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one aspect" or "an aspect" means that a particular element, feature, structure, or characteristic described in connection with the aspect is included in at least one aspect. The appearances of the phrase "in one aspect" in various places in the specification are not necessarily all referring to the same aspect.

Some aspects may be described using the expression "coupled" and "connected" along with their derivatives. For example, some aspects may be described using the term "coupled" to indicate that two or more elements are in direct physical or electrical contact. The term "coupled," however, may also mean that two or more elements are not in direct contact with each other, but yet still co-operate or interact with each other. The aspects are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the aspects herein. This is done merely for convenience and to give a general sense of the description. This description, and the claims that follow, should be read to include one or at least one and the singular also includes the plural unless it is obvious that it is meant otherwise.

This detailed description is to be construed as an example only and does not describe every possible aspect, as describing every possible aspect would be impractical, if not impossible. One could implement numerous alternate aspects, using either current technology or technology developed after the filing date of this application.

**Claims**

1.  A computer-implemented method for stratifying patient cancer risk using molecular data, comprising:

    receiving, via one or more processors, molecular data corresponding to a patient;
    processing, via one or more processors, the molecular data using a machine learning model to determine the patient's molecular data risk,

       wherein the machine learning model is trained using a patient training dataset and/or a reference training data set,
       wherein the machine learning model uses univariate gene selection, RNA bias correction and multivariate gene selection to filter and correct its training data, and
       wherein the machine learning model includes a survival model; and

    generating a matched treatment strategy corresponding to the patient based upon the patient's molecular data risk.

2. The computer-implemented method of claim 1, wherein the survival model is a Cox Proportional Hazards model.

3. The computer-implemented method of claim 1 or 2, wherein the molecular data corresponding to the patient includes RNA seq data.

4. The computer-implemented method of any one of claims 1 to 3, wherein the cancer is endometrial cancer, and wherein the machine learning model was trained on a cohort of patient data selected using a greedy algorithm.

5. The computer-implemented method of claim 4, wherein the greedy algorithm includes:

   identifying patients with uterine subtype and primary site endometrium or uterus;
   identifying time to progression eligible patients;
   identifying patients with sarcoma cancers;
   identifying patients having serous tissue cancers and squamous tissue cancers; and
   identifying patients having sarcoma cancers, serous tissue cancers and squamous tissue cancers.

6. The computer-implemented method of any one of claims 1 to 5, wherein the patient has a pre-existing clinical risk group assignment of at least one of the following: low clinical risk, low-intermediate clinical risk, high-intermediate clinical risk or high clinical risk.

7. The computer-implemented method of claim 6, wherein generating the treatment strategy corresponding to the patient based upon the patient's molecular data risk includes generating the treatment strategy based upon both of (i) the pre-existing clinical risk group; and (ii) the patient's molecular risk.

8. The computer-implemented method of claim 7, wherein the pre-existing clinical risk group is high-intermediate, the patient's molecular risk is high, and the treatment strategy is at least one of systemic therapy or external beam radiation therapy; and/or

   wherein the pre-existing clinical risk group is high-intermediate, the patient's molecular risk is low, and the matched treatment strategy is observation; and/or
   wherein the pre-existing clinical risk group is low-intermediate, the patient's molecular risk is high, and the matched treatment strategy is at least one of brachytherapy, external beam radiation therapy or systemic therapy; and/or
   wherein the pre-existing clinical risk group is high, the patient's molecular risk is low, and the matched treatment strategy is observation; and/or
   wherein the pre-existing clinical risk group is high, the patient's molecular risk is low, and the matched treatment strategy is observation; and/or
   wherein the pre-existing clinical risk group is high, the patient's molecular risk is high, and the matched treatment strategy is at least one of systemic therapy or external beam radiation therapy.

9. The computer-implemented method of any one of claims 1 to 8, wherein the matched treatment strategy includes at least one of systemic therapy, external beam radiation therapy, brachytherapy or observation.

10. A computing system for stratifying patient cancer risk using molecular data, comprising:

   one or more processors, and
   one or more memories having stored thereon computer-executable instructions that, when executed by the one or more processors, cause the computing system to:

      receive molecular data corresponding to a patient;
      process the molecular data using a machine learning model to determine the patient's molecular data risk,

         wherein the machine learning model is trained using a patient training dataset and a reference training data set,
         wherein the machine learning model uses univariate gene selection, RNA bias correction and multivariate gene selection to filter and correct its training data, and
         wherein the machine learning model includes a survival model; and

generate a matched treatment strategy corresponding to the patient based upon the patient's molecular data risk.

11. The computing system of claim 10, wherein the cancer is endometrial cancer, and wherein the machine learning model was trained on a cohort of patient data selected using a greedy algorithm.

12. The computing system of claim 11, wherein the memories have stored thereon instructions that, when executed, cause the computing system to:

identify patients with uterine subtype and primary site endometrium or uterus;
identify time to progression eligible patients;
identify patients with sarcoma cancers;
identify patients having serous tissue cancers and squamous tissue cancers; and
identify patients having sarcoma cancers, serous tissue cancers and squamous tissue cancers.

13. The computing system of claims 10 to 12, wherein the patient has a pre-existing clinical risk group assignment of at least one of the following: low clinical risk, low-intermediate clinical risk, high-intermediate clinical risk or high clinical risk.

14. A computer-readable medium having stored thereon computer-executable instructions that, when executed by one or more processors, cause a computer to:

receive molecular data corresponding to a patient;
process the molecular data using a machine learning model to determine the patient's molecular data risk,

wherein the machine learning model is trained using a patient training dataset and a reference training data set,
wherein the machine learning model uses univariate gene selection, RNA bias correction and multivariate gene selection to filter and correct its training data, and
wherein the machine learning model includes a survival model; and

generate a matched treatment strategy corresponding to the patient based upon the patient's molecular data risk.

15. The computer-readable medium of claim 14, wherein the patient has a pre-existing clinical risk group assignment of at least one of the following: low clinical risk, low-intermediate clinical risk, high-intermediate clinical risk or high clinical risk.

**FIG. 1**

200

**Training**

| 202a | 202b | 202c | 202e | 202f | 202g |

TCGA train → Univariate gene selection → RNA bias correction → Multiva gene selection → Train CoxPH model → Threshold Selection

Proprietary dataset — 202d

**FIG. 2**

300

# Cohort Selection

1. Tempus patients with uterine subtype and primary site either endometrium or uterus (baseline)

2. Keep RNA V1 patients only

3. Keep time to progression eligible patients only

4. Remove sarcomas using diagnosis_rollup

5. Remove serous and squamous using diagnosis_rollup

6. Remove sarcoma, serous, and squamous using diagnosis_final

N~=1200

N~=700

N~=250

N~=200

N~=170

N~=110

**FIG. 3**

FIG. 4

**FIG. 5A**

510

FIG. 5B

520

**FIG. 5C**

**FIG. 6A**

**FIG. 6B**

FIG. 6C

**FIG. 6D**

FIG. 6E

FIG. 6F

700

[patient name]

Diagnosis
Endometrial cancer

Accession No.

xEN

Date of Birth

Sex
**Female**

Physician
**Thomas**

Institution
**Test Data Institution**

**TEMPUS | ENDO**
**ENDOMETRIAL ALGO**

Tumor specimen:
Endometrium
Test Data Management Pathology Lab
Collected 1/2/2021
Received 5/4/2023
Tumor Percentage: 50%

**Notes**
The patient does not currently qualify for
any clinical trials in our database.

# MOLECULAR RISK

## Risk Group

MR-HIGH

This patient is predicted to be at high risk
of distant recurrence if treated with
brachytherapy or observation alone

## Risk Score

62

0     25     100

Risk group threshold

# 4-YEAR DISTANT RECURRENCE

31%

This patient is predicted to have a 31% risk of distant
recurrence over 4 years if treated with brachytherapy or
observation alone

# TCGA SUBTYPE

NSMP

**TEMPUS**   Electronically Signed By   CLIA Number   Date Signed/Reported   Laboratory Medical Director   Tempus ID #   Pipeline Version   1/6
parth qa       09/14/2023       CAP       3.10.0

Tempus Labs, Inc • 600 West Chicago Avenue, Ste 510 • Chicago, IL • 60654 • tempus.com • support@tempus.com

## FIG. 7

| | Europäisches Patentamt European Patent Office Office européen des brevets | **EUROPEAN SEARCH REPORT** | **Application Number** EP 24 21 3383 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LV HUANG ET AL: "Bioinformatics-based analysis of programmed cell death pathway and key prognostic genes in gastric cancer: Implications for the development of therapeutics", THE JOURNAL OF GENE MEDICINE, JOHN WILEY & SONS, INC, US, vol. 26, no. 1, 5 September 2023 (2023-09-05), page n/a, XP072576550, ISSN: 1099-498X, DOI: 10.1002/JGM.3590 * the whole document * | 1-15 | INV. G16B20/00 G16B40/20 |
| X | FAN WANG ET AL: "Predictors of breast cancer cell types and their prognostic power in breast cancer patients", BMC GENOMICS, BIOMED CENTRAL LTD, LONDON, UK, vol. 19, no. 1, 13 February 2018 (2018-02-13), pages 1-13, XP021253418, DOI: 10.1186/S12864-018-4527-Y * the whole document * | 1-15 | |

- - - - -

-/--

| | | TECHNICAL FIELDS SEARCHED (IPC) |
|---|---|---|
| | | G16B |

The present search report has been drawn up for all claims

| Place of search | Date of completion of the search | Examiner |
|---|---|---|
| Munich | 26 March 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 21 3383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GONZALEZ BOSQUET JESUS ET AL: "Creation and validation of models to predict response to primary treatment in serous ovarian cancer", SCIENTIFIC REPORTS, vol. 11, no. 1, 1 January 2021 (2021-01-01), pages 1-14, XP093206222, US ISSN: 2045-2322, DOI: 10.1038/s41598-021-85256-9 Retrieved from the Internet: URL:https://www.nature.com/articles/s41598-021-85256-9> * the whole document * | 1-15 | |
| X | HUANG ZHEN-DONG ET AL: "Construction of Prognostic Risk Prediction Model of Oral Squamous Cell Carcinoma Based on Nine Survival-Associated Metabolic Genes", FRONTIERS IN PHYSIOLOGY, vol. 12, 16 March 2021 (2021-03-16), XP093263199, CH ISSN: 1664-042X, DOI: 10.3389/fphys.2021.609770 Retrieved from the Internet: URL:https://pmc.ncbi.nlm.nih.gov/articles/PMC8011568/pdf/fphys-12-609770.pdf> * the whole document * | 1-15 | |
| A | US 2022/101952 A1 (AHMED TALAL [US] ET AL) 31 March 2022 (2022-03-31) * paragraphs [0077] - [0278] * | 1-15 | TECHNICAL FIELDS SEARCHED (IPC) |

The present search report has been drawn up for all claims

| Place of search | Date of completion of the search | Examiner |
|---|---|---|
| Munich | 26 March 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 3383

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2025

| Patent document cited in search report | Publication date | Patent family member(s) | Publication date |
|---|---|---|---|
| US 2022101952 A1 | 31-03-2022 | US 2022059190 A1 | 24-02-2022 |
| | | US 2022101951 A1 | 31-03-2022 |
| | | US 2022101952 A1 | 31-03-2022 |
| | | WO 2022040688 A1 | 24-02-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63599471
- US 40502521

- US 54811821
- US 54808421

**Non-patent literature cited in the description**

- **HENRY M KEYS et al.** A phase III trial of surgery with or without adjunctive external pelvic radiation therapy in intermediate risk endometrioid endometrial adenocarcinoma: a Gynecologic Oncology Group study. *Gynecologic Oncology*, 2004, vol. 92 (3), 744-751, https://doi.org/10.1016/j.ygyno.2003.11.048
- **B. G. WORTMAN et al.** Ten-year results of the PORTEC-2 trial for high-intermediate risk endometrial carcinoma: improving patient selection for adjuvant therapy. *Nature, British Journal of Cancer*, 2018, vol. 119, 1067-1074, https://doi.org/10.1038/s41416-18-0310-8

- **SCHOLTEN et al.** Postoperative radiotherapy for Stage 1 endometrial carcinoma: Long-term outcome of the randomized PORTEC trial with central pathology review. *International Journal of Radiation Oncology*Biology*Physics*, 2005, vol. 63 (3), 834-838, https://doi.org/10.1016/j.ijrobp.2005.03.007